# EUROPEAN PATENT SPECIFICATION

(11) **EP 0 006 722 B1**
(45) Date of publication and mention of the grant of the patent: **05.09.1984**
(21) Application number: 79301151.1
(22) Date of filing: 15.06.1979
(51) Int. Cl.: C07D 405/14, A01N 43/28, A01N 43/50, A01N 43/60, A01N 43/64, A01N 43/84, A61K 31/335, A61K 31/41, A61K 31/415, A61K 31/495, A61K 31/535

(54) **Derivatives of (4-(piperazin-1-yl-phenyloxymethyl)-1.3-dioxolan-2-ylmethyl)-1H-imidazoles and -1H-1.2.4-triazoles, their preparation and use as fungicides and bactericides**
(4-(Piperazin-1-yl-phenyloxymethyl)-1.3-dioxolan-2-ylmethyl)-1H-imidazol- und -1H-1.2.4-triazol-Derivate, ihre Herstellung und ihre Verwendung als Fungizide und Bacterizide
Dérivés de (4-(pipérazin-1-yl-phényloxyméthyl)-1.3-dioxolan-2-ylméthyl)-1H-imidazoles et -1H-1.2.4-triazoles, leur préparation et leur utilisation comme fongicides et bactéricides

(30) Priority: 03.07.1978 US 921380; 26.03.1979 US 23807
(43) Date of publication of application: 09.01.1980
(73) Proprietor: JANSSEN PHARMACEUTICA N.V., 2340 Beerse (BE)
(72) Inventor: Heeres, Jan, B-2350 Vosselaar (BE); Mostmans, Joseph, B-2000 Antwerp (BE)
(74) Representative: Grundy, Derek George Ritchie

## Description

### Background of the invention

In U.S. Pat. No. 3,936,470 and Belg. Pat. No. 837,831 there are described a number of 1-(1,3-dioxolan-2-ylmethyl)-lH-imidazoles and lH-1,2,4-triazoles having antifungal and antibacterial properties. The compounds of this invention differ from the foregoing essentially by the substitution of the aryloxy-moiety with a piperazinyl group, substituted in the 4-position with an aliphatic group or a sulfonyl group. Other similar compounds are described in U.S. Pat. No. 4,144,346. The compounds of this invention differ from the latter essentially by the nature of the Y-substituent on the piperazine nitrogen atom.

Other similar imidazoles and 1 H-1,2,4-triazoles having antifungal and antibacterial properties are disclosed in DE-A-2803870.

### Description of the invention

This invention is concerned with novel 1 H-imidazole and 1 H-1 ,2,4-triazole derivatives which may structurally be represented by the formula: 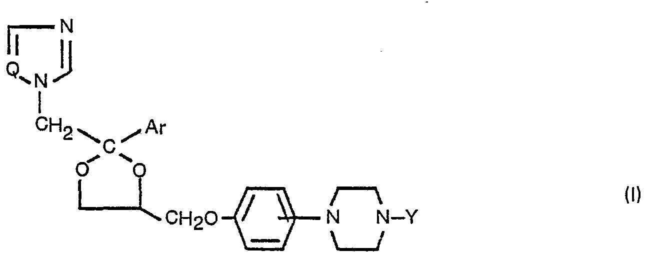 and the pharmaceutically acceptable acid addition salts and stereochemically isomeric forms thereof, wherein:
Q is a member selected from the group consisting of N and CH;

Ar is a member selected from the group consisting of phenyl, thienyl, halothienyl and substituted phenyl, said substituted phenyl having from 1 to 3 substituents each independently selected from the group consisting of halo, C₁―C₆ alkyl, C₁―C₆ alkyloxy and trifluoromethyl; and the radical Y is a member selected from the group consisting of:
a radical of the formula 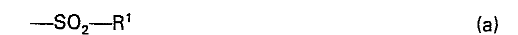 wherein R' is selected from the group consisting of trifluoromethyl and aryl;
a radical of formula 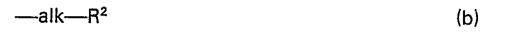 wherein alk is a member selected from the group consisting of C₁―C₆ alkylene and C₂―C₆ alkenylene and R² is a member selected from the group consisting of cyano, amino, mono- and di(C₁―C₆ alkyl)amino, arylamino, mono- and di(aryl C₁―C₆ alkyl)amino, 1-pyrrolidinyl, 1-morpholinyl, 1-piperidinyl, aryloxy and aryl, provided that alk is other than methylene when R² is phenyl;
a radical of formula 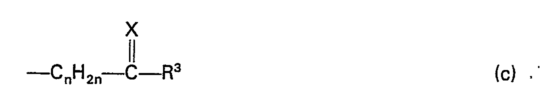 wherein n is an integer of from 0 to 6 inclusive, X is 0 or S and R³ is selected from the group consisting of hydrogen, mono-, di- and trihalo-C₁―C₆-alkyl, amino, mono- and di(C₁―C₆ alkyl)amino, arylamino, mono- and di(aryl C₁―C₆ alkyl)amino, amino C₁―C₆ alkyl, mono- and di(C₁―C₆ alkyl)amino C₁―C₆ alkyl, (1-pyrrolidinyl) C₁―C₆ alkyl, (1-morpholinyl) C₁―C₆ alkyl, (1-piperidinyl) C₁―C₆ alkyl, aryl, aryl C₁―C₆ alkyl, aryl C₂―C₆ alkenyl and C₁―C₆ alkyloxycarbonyl C₁―C₆ alkyloxy, provided that:
   i) said n is other than 0 or 1 when said R³ is amino or C₁―C₆ alkylamino; and
   ii) said n is other than 0 when said R³ is hydrogen di(C₁―C₆ alkyl)amino or aryl; and
a radical of formula 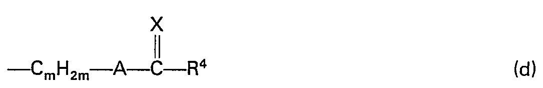 wherein m is an integer of from 1 to 6 inclusive, A is 0 or NH, X is 0 or S and R⁴ is a member selected from the group consisting of hydrogen, C₁―C₆ alkyl, C₁―C₆ alkyloxy, aryl, aryloxy, aryl C₁―C₆ alkyl, amino, mono- and di(C₁―C₆ alkyl)amino, arylamino, mono- and di(aryl C₁―C₆ alkyl)amino, 1-pyrrolidinyl, 1-morpholinyl and 1-piperidinyl;

wherein said aryl, as used in the foregoing definitions, is selected from the group consisting of phenyl, substituted phenyl, thienyl, halothienyl, C₁―C₆ alkylthienyl and pyridinyl, said substituted phenyl having from 1 to 3 substituents each independently selected from the group consisting of C₁―C₆ alkyl, C₁―C₆ alkyloxy, halo, amino, mono- and di(C₁―C₆ alkyl)amino, C₁―C₆ alkylcarbonylamino, nitro and trifluoromethyl.

The preferred compounds of this invention are those wherein the 1-piperazinyl group is attached to the 4-position of the phenoxymethyl moiety.

As used herein the term "halo" is generic to halogens of atomic weight less than 127, i.e., fluoro, chloro, bromo and iodo; the term "C₁―C₆ alkyl" denotes straight and branch chained hydrocarbon radicals having from 1 to 6 carbon atoms such as methyl, ethyl, 1-methylethyl, 1,1-dimethylethyl, propyl, butyl, pentyl or hexyl and the term "C₂―C₆ alkenyl" denotes straight and branched alkenyl radicals having from 2 to 6 carbon atoms such as ethenyl, 2-propenyl, 3-butenyl or 3-methyl-2-pentenyl.

In order to simplify the structural representation of compounds (I) and of certain starting materials and intermediates used in the preparation thereof, the 2-Ar-2-(1H-imidazol-1-yl-methyl or 1H-1,2,4-triazol-1-ylmethyl)-1,3-dioxolan-4-yl group wherein Ar is as previously defined, will hereafter be represented by the symbol D:

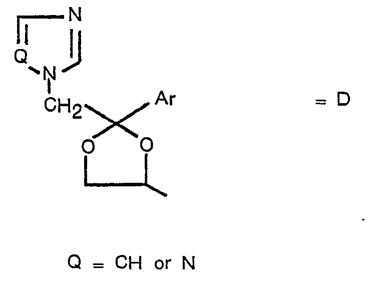

The compounds of formula (I) wherein Y has the previously defined meaning, can generally be prepared by O-alkylating an appropriately substituted phenol of formula (III) with an appropriate reactive ester of formula (II), following standard O-alkylation procedures

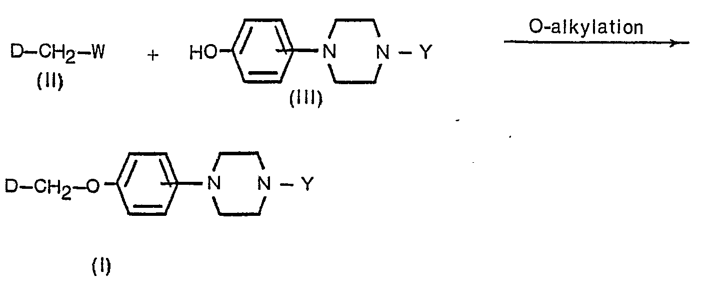

In formula (II), W has the meaning of a reactive ester residue such as, for example, halo, methyl- sulfonyloxy, 4-methylphenylsulfonyloxy and the like. The reaction of (II) with (III) (s carried out under art-known conditions of performing O-alkylations with reactive esters. The reaction is generally carried out in an appropriate reaction-inert organic solvent, such as N,N-dimethylformamide, N,N-dimethylacetamide, hexamethylphosphoric triamide, dimethylsulfoxide or 4-methyl-2-pentanone, optionally in admixture with other reaction inert solvents such as aromatic hydrocarbons, e.g., benzene, methylbenzene or dimethylbenzene. Further it is advantageous to add to the reaction mixture an appropriate base such as an alkali metal hydride or carbonate, in order to enhance the rate of the reaction. Otherwise it may be advantageous to first convert the substituted phenol (III) into a metal salt thereof, preferably the sodium salt, in the usual manner, e.g., by the reaction of (III) with metal bases such as sodium hydride or sodium hydroxide and to use thereafter said metal salt in the rection with (II). Somewhat elevated temperatures are appropriate to enhance the reaction rate and most preferably the reaction is carried out at from 80°C to 130°C.

The compounds of formula (I) may also be prepared by the reaction of an appropriate piperazine (IV) with an appropriate reactant of formula (V), wherein W has the meaning of a reactive ester, as previously decribed.

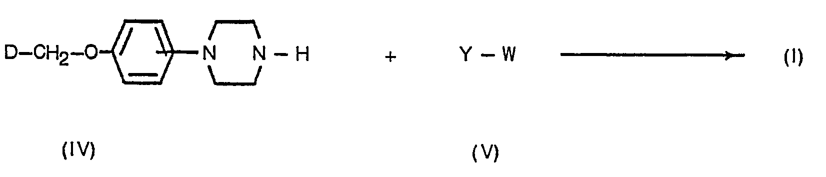

In said reaction the meaning of W as well as the reaction-conditions depend upon the nature of Y as will be explained hereinafter.

In case Y is other than a radical of formula (a), other than a radical of formula (c) wherein n is 0 and other than a radical of formula (c) wherein n is 1 and R³ is Cₗ-C₆ alkyloxycarbonyl Cₗ-C₆ alkyloxy, W may be halo, or a sulfonyloxy group and the reaction is then preferably conducted in a suitable reaction-inert solvent such as a lower alkanol such as, ethanol or butanol; an amide such as, N,N-dimethylformamide; a ketone such as, 4-methyl-2-pentanone; dimethylsulfoxide; a halogenated hydrocarbon such as, dichloromethane. Said solvents may be used as such or in admixture with other reaction-inert solvent such as, aromatic and aliphatic hydrocarbons, such as, hexane or benzene. Somewhat elevated temperatures may be advantageous to enhance the rate of the reaction and, most preferably, the reaction is carried out at from 80°C to 130°C. Further it may be advantageous to add an appropriate base such as, an amine such as N,N-diethylethanamine, or pyridine; an alkali metal- or an earth alkaline metal carbonate or hydrogen carbonate, such as sodium carbonate or potassium hydrogen carbonate.

In a particular case, when W represents a very reactive leaving group such as halo in a-position of a carbonyl function, and when carbon dioxide or a carbonate or a hydrogen carbonate is present in the reaction medium, there may occur an insertion-reaction of said carbon dioxide. In such manner the compounds of formula (I) wherein Y represents a radical of formula (c) wherein n is 1 and R³ is a C₁―C₆ alkyloxycarbonyl C,-Cₛ alkyloxy radical may be prepared by the reaction of an appropriate piperazine of formula (IV) with a reagent of formula (V) wherein Y is C₁―C₆ alkyloxycarbonyl C₁―C₆ alkyl in the presence of carbon dioxide or a suitable carbonate or hydrogen carbonate.

In case Y represents a radical of formula (a) or a radical of formula (c) wherein n is 0, then W is selected from the group consisting of halo, most preferably chloro, and the reaction is preferably conducted in a suitable reaction-inert solvent such as, an aliphatic-, an alicyclic- or an aromatic hydrocarbon, such as hexane, cyclohexane or benzene; a halogenated hydrocarbon, such as trichloromethane. More particularly, when Y is a radical of formula (a), the solvent may even so be selected from the group consisting of water; a lower alkanol, such as ethanol and an amide, such as dimethylacetamide.

The compounds of formula (I) wherein Y represents a radical (b) wherein alk is as previously defined and wherein R² is a previously defined, but other than cyano and other than aryl, said R² being represented by R²ₐ and said compounds by the formula (I-a), may be prepared by substituting the reactive leaving group W in an intermediate of formula (VI) by an appropriately substituted amine or alcohol of formula (VII), following art-known 0-, respectively N-alkylation procedures as previously described herein.

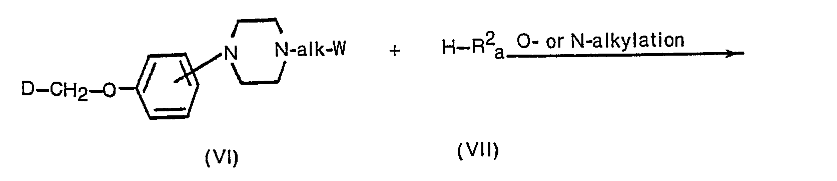

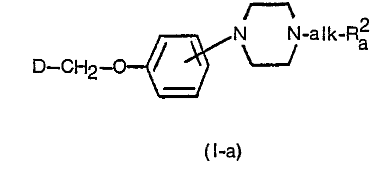

The compounds of formula (I-a) wherein R²ₐ is amino can more preferably be prepared by reacting (VI) with ammonia wherein 2 protons have been replaced by protective groups, such as di(phenylmethyl)amine or 1H-isoindo)e-1,3(2H)-dione, and subsequently eliminating the protective groups following art-known procedures, depending upon the nature of said protecting groups, such as by catalytic hydrogenation or by alkaline hydrolysis.

Said primary derivatives of formula (I-a) may also be derived from the corresponding cyanides by reducing the latters following art-known cyano-to-amine reduction procedures such as, catalytic hydrogenation.

The compounds of formula (I-a) wherein R²ₐ is amino, mono- and di(C₁―C₆ alkyl)amino, arylamino, mono- and di(aryl C₁―C₆ alkyl)amino, 1-pyrrolidinyl, 1-morpholinyl or 1-piperidinyl may also be prepared starting from an appropriate carbonyl compound and ammonia or an appropriately substituted primary or secondary amine following art-known reductive amination procedures, such as by catalytically hydrogenating the starting materials in the presence of an appropriate catalyst such as platina-on-charcoal.

The compounds of formula (I) wherein Y represents a radical (d) wherein A and R⁴ are as previously defined and wherein X is 0, said compounds being represented by he formula (I-b) may easily be prepared by the reaction of an amine or an alcohol of formula (VIII) with an appropriately substituted carboxylic acid of formula 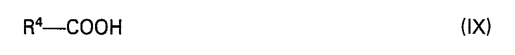 or a functional derivative thereof, most desirable, its acyl halide 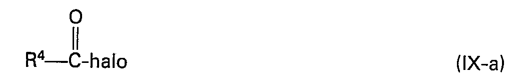 or its anhydride

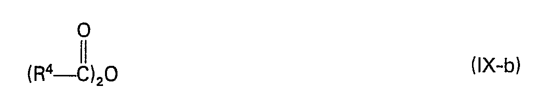

The reaction may be carried out by stirring and heating the reactants together in the presence of a suitable reaction-inert solvent such as, a halogenated hydrocarbon, such as, dichloromethane.

When an acyl halide of formula (IX-a) is used, it may be advantageous to add a base, such as, an amine, e.g., N,N-diethylethanamine and the like, in order to neutralize the liberated hydrohalic acid.

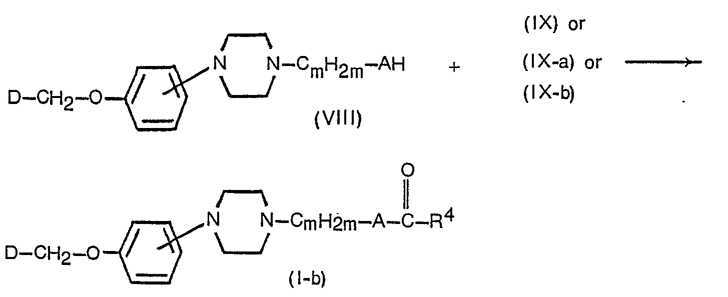

The compounds of formula (I) wherein Y represents a radical (d) wherein A and X are as previously defined and R⁴ is amino, C₁―C₆ alkylamino, arylamino or aryl C₁―C₆ alkylamino, said R⁴ being represented by -NHR⁴ₐ and said compounds by the formula (I-c), may conveniently be prepared by the addition-reaction of an appropriate amine or alcohol of formula (VIII) with an appropriately substituted isocyanate or isothiocyanate of formula (X).

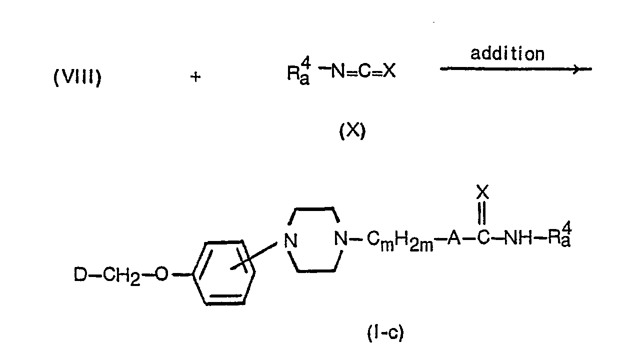

The addition-reaction is preferably carried out by stirring and heating the reactants together in the presence of a suitable reaction-inert solvent such as the aromatic hydrocarbon methylbenzene; an halogenated hydrocarbon such as dichloromethane or the solvent acetonitrile. In order to enhance the rate of the reaction it may be advantageous to add an amine, such as diethyl ethanamine, to the reaction mixture.

Furthermore certain compounds of formula (I) may be derived from other compounds of formula (I) by art-known functional group transformations, e.g., the compounds of formula (I) wherein Y represents a radical (a) wherein R' is aminophenyl may be derived from the corresponding Cₗ-C₆ alkylcarbonylamino derivatives by alkaline hydrolysis.

The imidazole derivatives of formula (I), obtained in base form in the foregoing preparations, may be converted to their therapeutically useful acid addition salts by reaction with an appropriate acid, as, for example, inorganic acid such as hydrohalic acid, such as hydrochloric, hydrobromic or hydroiodic acid; sulfuric, nitric or thiocyanic acid; a phosphoric acid; an organic acid such as acetic, propanoic, hydroxyacetic, 2-hydroxypropanoic, 2-oxopropanoic, ethanedioic, propanedioic, 1,4-butanedioic, (Z)-2-butenedioic, (E)-2-butenedioic, 2-hydroxy-1,4-butanedioic, 2,3-dihydroxy-1,4-butanedioic, 2-hydroxy-1,2,3-propanetricarboxyiic, benzoic, 3-phenyl-2-propenoic, a-hydroxybenzeneacetic, methanesulfonic, ethanesulfonic, 2-hydroxyethanesulfonic, 4-methylbenzenesulfonic, 2-hydroxybenzoic, 4-amino-2-hydroxybenzoic, 2-phenoxybenzoic or 2-acetyloxybenzoic acid. The salts are in turn converted to the corresponding free bases in the usual manner, such as by reaction with alkali such as sodium or potassium hydroxide.

A number of the intermediates and starting materials used in the foregoing preparations are known compounds, others may be prepared according to art-known methodologies of preparing similar compounds and some of them are novel and consequently their preparation will be described hereafter.

The intermediates of formula (III) can generally be derived from the corresponding methoxy derivatives (XI) by converting the latter into the corresponding phenols (III) using, e.g., a strong non- oxidizing acid such as, hydrobromic acid in glacial acetic acid.

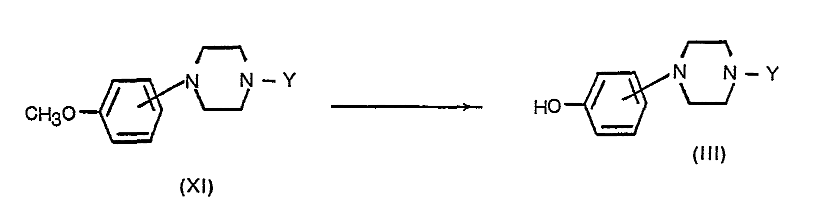

The intermediates of formula (XI) used as starting materials herein, can be obtained by N-substituting an appropriate piperazine (XII) with a reactant of the formula (V) wherein W is a reactive leaving group, as previously defined.

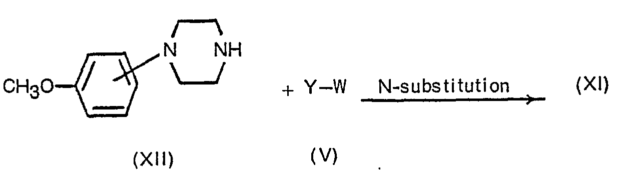

The intermediates of formula (III) can alternatively be prepared by the reaction of an appropriate piperazine (XII-a) with a reactant of the formula (V), following the previously described procedure herein for the synthesis of (I) starting from (IV) and (V).

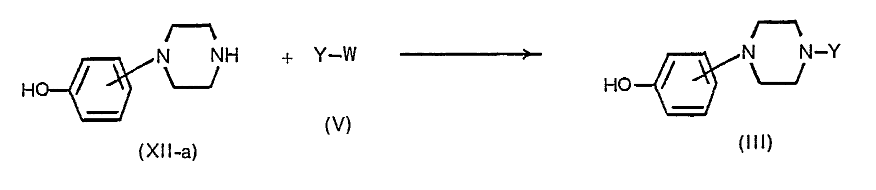

The intermediates of formula (IV) can generally be prepared by 0-alkylating an appropriately substituted phenol (XII-b), wherein P represents a protective group, with an appropriate reactive ester (II), following standard O-alkylation procedures, as previously described herein for the synthesis of (I) starting from (II) and (III), and subsequently eliminating the protective gorup of the thus obtained (XIII), following art-known procedures.

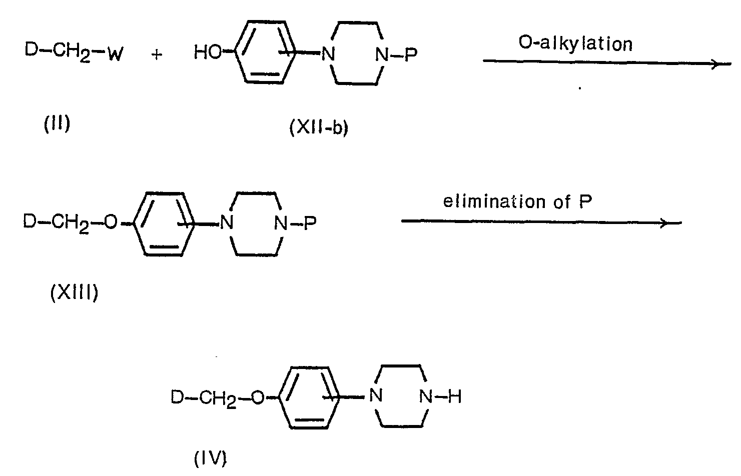

The intermediates of formula (VIII) can generally be derived from an intermediate (IV) by alkylating the latter with an appropriate reactive ester of formula (XIV), wherein m and W are as previously defined, following standard N-alkylating procedures as previously described herein for the synthesis of (I) starting from (IV) and (V).

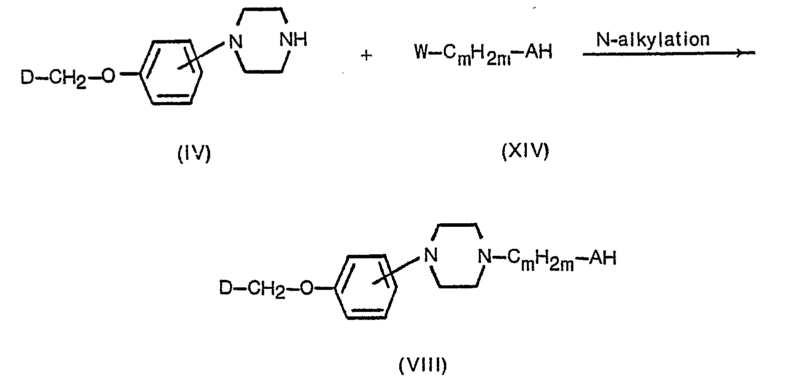

When m in formula (VIII) is 2, the same intermediates (VIII) may also be prepared by the reaction of (IV) with oxirane, respectively aziridine, e.g., by bubbling them through a heated solution of (IV) in a suitable organic solvent such as the lower alkanols, methanol, ethanol or 2-propanol.

Following the same procedure the intermediates of formula (VI) may be derived from an intermediate (IV) by N-alkylating the latter with an appropriate reactive ester of formula (XV) wherein W, and alk are as previously defined.

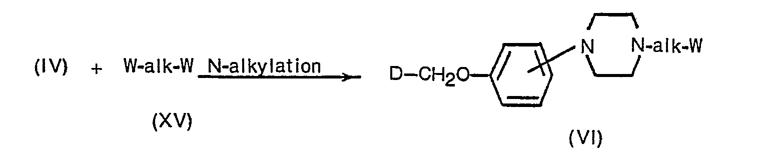

Starting materials of formula (11) wherein Q stands for CH and methods of preparing the same are described in U.S. Pat. No. 4,144,346. In general the reactive ester of formula (II) can be prepared along the following sequence of reactions.

An appropriate 1-Ar-2-bromoethanone of formula (XVI) is subjected to a ketalization reaction with 1,2,3-propanetriol following methodologies analogous to those described in Synthesis, 1974, (I) 23.

In a preferred manner of carrying out the reaction both reactants are refluxed together for several hours with azeotropic water removal in an appropriate organic solvent, preferably in the presence of a simple alcohol, such as, ethanol, propanol, butanol or pentanol, and in the presence of an appropriate strong acid such as 4-methylbenzenesulfonic acid. Suitable organic solvents are, aromatic hydrocarbons, such as benzene, methylbenzene or dimethylbenzene and saturated hydrocarbons, such as hexane.

The thus obtained dioxolane (XVII) is then reacted with benzoyl chloride to obtain a benzoate of the formula (XVIII) and the latter is subsequently reacted with 1 H-imidazole or 1 H-1,2,4-triazole.

Said reaction is preferably carried out by stirring and heating the reactants together in a suitable organic solvent, such as N,N-dimethylformamide, in the presence of an appropriate strong metal base, such as sodium methanolate, to obtain an intermediate of the formula (XIX).

The desired reactive esters of formula (II) are then conveniently prepared by first hydrolyzing (XIX) in alkaline medium and thereafter converting the hydroxy group of the thus obtained (XX) into a reactive ester thereof according to methodologies generally known in the art.

For example, methanesulfonates and 4-methylbenzenesulfonates are conveniently prepared by the reaction of the alcohol with methanesulfonyl chloride or 4-methylbenzenesulfonyl chloride and halides may be prepared by the reaction of the alcohol with an appropriate halogenating agent usch as, thionyl chloride, sulfuryl chloride, phosphor pentachloride, phosphor pentabromide or phosphoryl chloride.

When the reactive ester is an iodide, it is preferably derived from the corresponding chloride or bromide by the replacement of that halogen with iodine.

The foregoing reactions may be illustrated as follows:

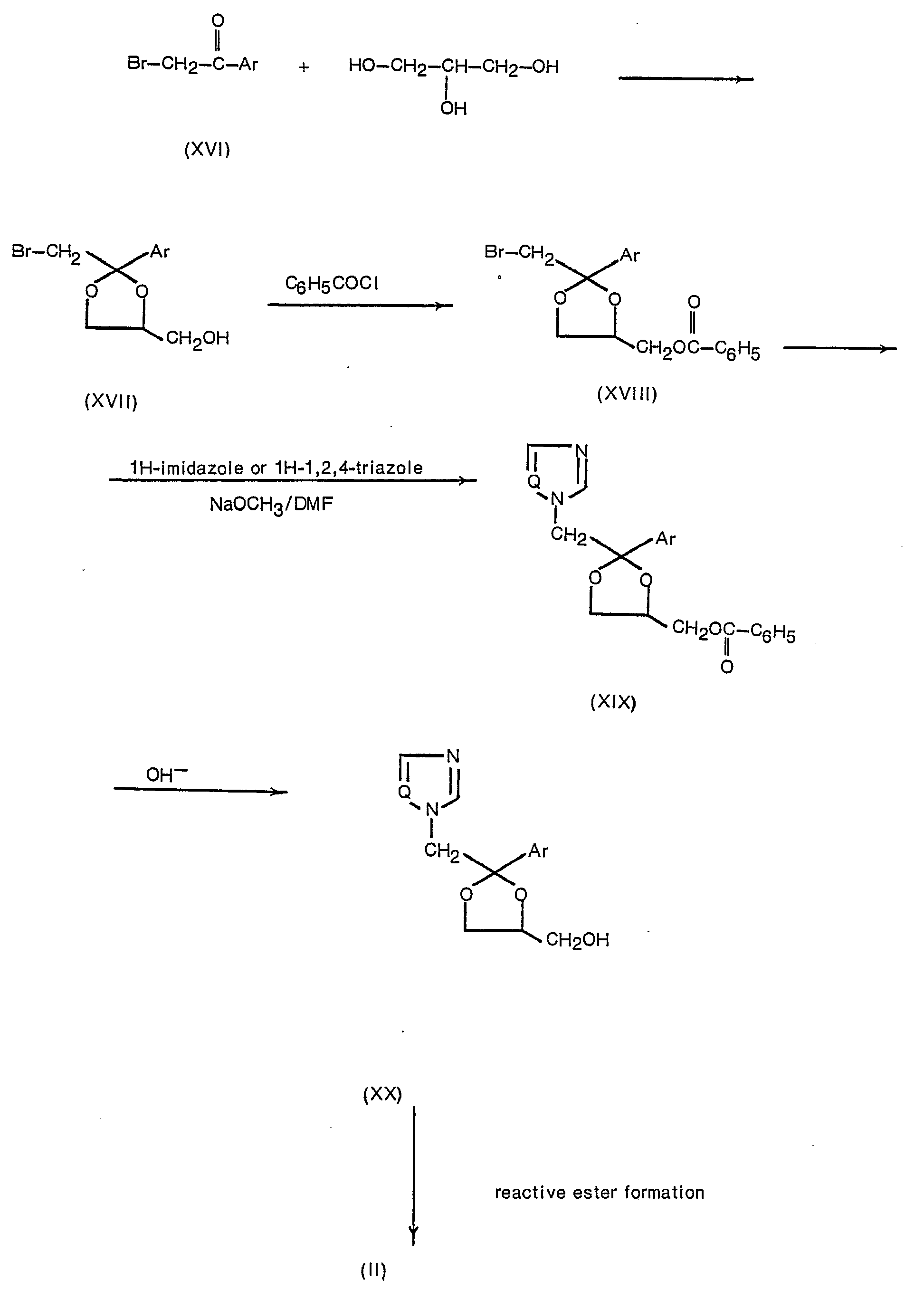

From formula (I) it is evident that the compounds of this invention have at least two asymmetric carbon atoms in their structures, namely those located in the 2- and 4- positions of the dioxolane nucleus, and consequently they can exist under different stereochemically isomeric forms. The stereochemically isomeric forms of (I) and the pharmaceutically acceptable acid addition salts thereof are intended to be within the scope of this invention.

The diastereomeric racemates of (I), denoted as cis and trans forms respectively, according to the rules described in C.A., 76, Index Guide, Section IV, p. 85 (1972), may be obtained separately by conventional methods. Appropriate methods which may advantageously be employed therefore include, selective crystallization and chromatographic separation, such as column-chromatography.

Since the stereochemical configuration is already fixed in the intermediates (II) and (IV) it is also possible to separate cis and trans forms at this or even an earlier stage, whereupon the corresponding forms of (I) may be derived therefrom in the previously indicated manner. The separation of cis and trans forms of such intermediates may be performed by conventional methods as described hereabove for the separation of cis and trans forms of the compounds (I).

It is evident that the cis and trans diastereomeric racemates may be further resolved into their optical isomers, cis(+), cis(-), trans(+) and trans(-) by the application of methodologies known to' those skilled in the art.

The compounds of formula (I) and pharmaceutically acceptable acid addition salts thereof are useful agents in combatting fungi and bacteria. For example, said compounds and acid addition salts thereof were found to be highly active against a wide variety of fungi such as, Microsporum canis, Ctenomyces mentagrophytes, Trichophyton rubrum, Phialophora verrucosa, Cryptococcus neoformans, Candida tropicalis, Candida albicans, Mucor species, Aspergillus fumigatus, Sporotricum schenckii and Saprolegnia species, and against bacteria such as, Erysipelotrix insidiosa, Staphylococci such as Staphylococcus hemolyticus and Streptococci such as Streptococcus pyrogenes. In view of their potent, local as well as systemic, antimicrobial activity the compounds of this invention constitute useful tools for the destruction or prevention of the growth of fungi and bacteria and more particularly they can effectively be used in the treatment of subjects suffering from such microorganism.

The strong antimicrobial activity of the compounds (I) is clearly evidenced by the data obtained in the following experiments, which data is only given to illustrate the useful antimicrobial properties of all the compounds (I).

### Experiment A

### Activity of compounds (I) against vaginal candidosis in rats

Female Wistar rats of ±100 g body weight are used. They are ovariectomized and hysterectomized and after three weeks of recovery, 100 µg of oestradiol undecylate in sesame oil is given subcutaneously once a week for 3 consecutive weeks. The thus induced pseudooestrus is controlled by microscopic examination of vaginal smears. Food and water are left available ad libitum. The rats are infected intravaginally with 8.101 cells of Candida albicans, grown on Sabouraud broth for 48 hours at 37°C and diluted with saline. The date of infection varies from day +25 to day +32 after surgical intervention, depending on the appearance of signs of induced pseudooestrus.

The drugs under investigation are administered orally once a day for two days starting from the day of infection. For each experiment there are placebo treated controls. The results are assessed by taking vaginal smears with sterile swabs on several days after the infection. The swabs are put into Sabouraud broth in petri-dishes and incubated for 48 hours at 37°C. If no growth of Candida albicans occurs, i.e., when the animals are negative at the end of the experiment, this is due to drug administration because it never happens in placebo treated controls.

The table below gives the lowest oral dose of the drug under investigation which is found active at the 14th day after infection.

### Experiment B

### Activity of compounds (I) against crop candidosis in turkeys

Turkeys of 14 days old are infected in the crop with 4.1µ° Candida albicans cells, grown on Sabouaud broth for 48 hours at 37°C and diluted with saline. The volume of the inoculum is 1 ml. The drugs under investigation are premixed in 500 mg of lacton and thereafter admixed in 1000 g of meal without any additives. The concentration of the drug under investigation in the meal is expressed in mg/kg.

The animals are given the medicated feed for 13 consecutive days starting on the day of infection. At the end of the experiment all animals are sacrificed. At autopsy the crops are removed, emptied and grinding in an ultra-turrax mixer in 15 ml of sterile saline. Colony counting is done on Sabouraud agar and the results given in the table represents the ED_{SO}, i.e., the dose of the drug whereby the crops of 50% of the animals are completely negative for Candida albicans.

The compounds listed in the table are intended to illustrate the present invention.

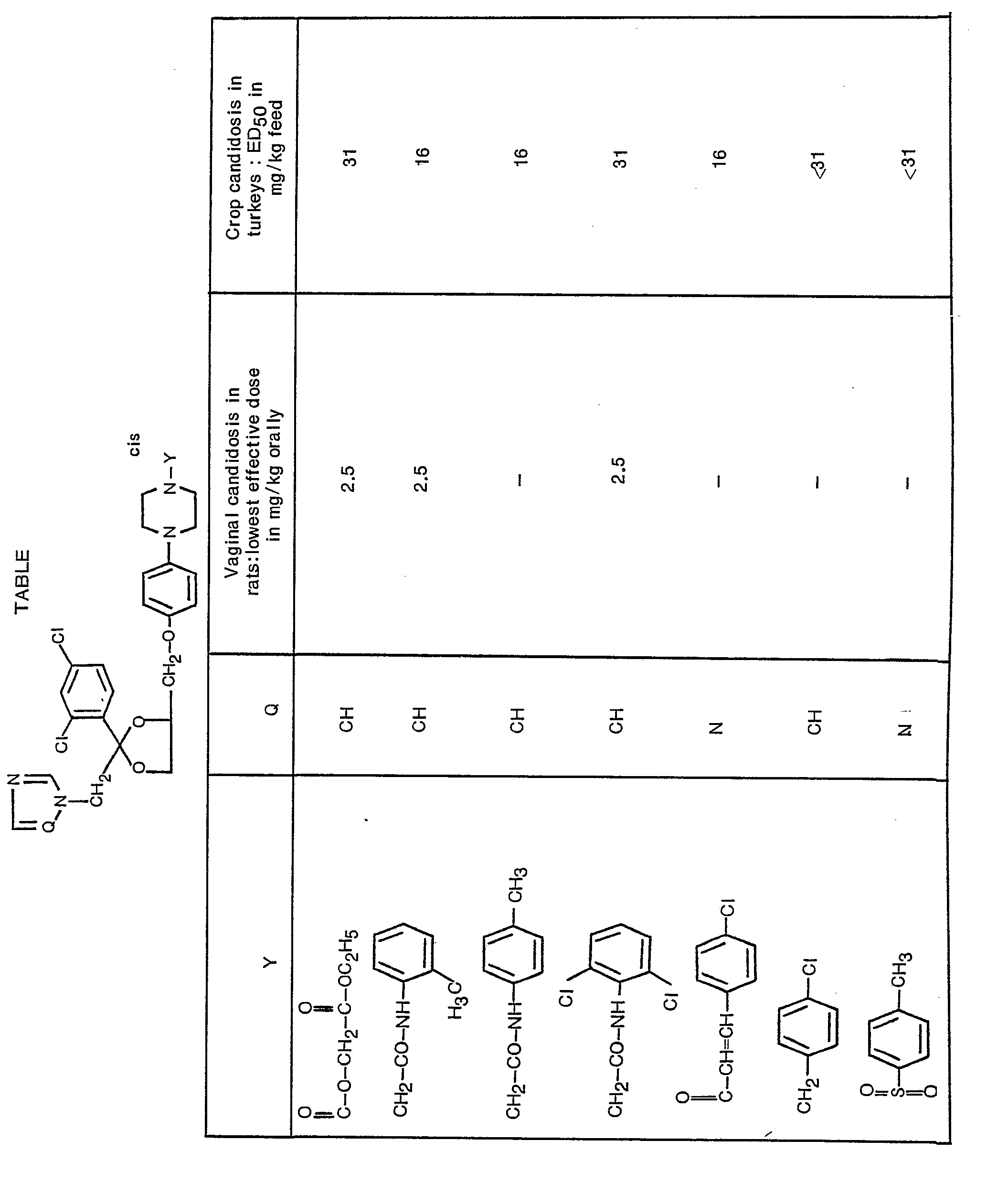

In view of their antifungal and antibacterial properties this invention provides valuable compositions comprising the subject compounds of formula (I) or acid addition salts thereof as the active ingredient in a solvent or a solid, semi-solid or liquid diluent or carrier. The compounds and compositions are effective in combatting fungal or bacterial growth by use of an effective antifungal or antibacterial amount of such compounds (I) or salts thereof. Antifungal and antibacterial compositions comprising an effective amount of an active compound (I), either alone or in combination with other active therapeutic ingredients, in admixture with suitable carriers may be readily prepared according to conventional pharmaceutical techniques for the usual routes of administration.

Preferred compositions are in dose unit form, comprising per dosage unit an effective quantity of the active ingredient in admixture with suitable carriers. Although the amount of the active ingredient per unit dosage may vary within rather wide limits, dosage units comprising from about 50 to about 500 mg and more particularly from about 100 to about 250 mg of the active ingredients are preferred.

The following examples are intended to illustrate the present invention.

Unless otherwise stated all parts therein are by weight.

### A. Preparation of intermediates

### Example I

To a stirred mixture of 15.5 parts of 4-(1-piperazinyl)phenol dihydrobromide, 40 parts of ethanol and 75 parts of water are added 15.2 parts of 4-methylbenzenesulfonyl chloride and stirring is continued for 30 minutes at room temperature. Then there are added portionwise 12.6 parts of sodium hydrogen carbonate at 0°C. Upon completion, stirring is continued overnight at room temperature. The precipitated product is filtered off and taken up in alkaline water. The mixture is filtered over hyflo and the filtrate is acidified with acetic acid. The precipitated product is filtered off and taken up in water. The free base is liberated in the conventional manner with a sodium hydroxide solution. The whole is filtered over hyflo and the filtrate is acidified with acetic acid. The precipitated product is filtered off and dried, yielding 4.8 parts of 1-(4-hydroxyphenyl)-4-(4-methylphenylsulfonyl)piperazine; mp. 193.1°C.

### Example II

To a stirred mixture of 34 parts of 4-(1-piperazinyl)phenol dihydrobromide, 100 parts of water and 130 parts of dichloromethane are added portionwise 42 parts of sodium hydrogen carbonate. Then there is added dropwise (slowly) a solution of 19 parts of chloroacetic acid anhydride in dichloromethane. Upon completion, stirring is continued for 1 hour at room temperature. The precipitated product is filtered off and crystallized from 4-methyl-2-pentanone, yielding 11 parts of 1-(2-chloroacetyl)-4-(4-hydroxyphenyl)piperazine.

A mixture of 10 parts of 1-(2-chloroacetyl)-4-(4-hydroxyphenyl)piperazine, 6.9 parts of morpholine and 40 parts of methanol is stirred and heated for 2 hours at 50°C. The reaction mixture is evaporated and the residue is stirred with water. The precipitated product is filtered off, dried on the filter and crystallized from 2₋propanol, yielding 5.5 parts of 1-(4-hydroxyphenyl)-4-[(4-morpholinyl)-acetyl]piperazine; mp. 197.3°C.

### Example III

A mixture of 3.4 parts of methanesulfonyl chloride and 100 parts of pyridine is stirred till all solid enters solution. Then there are added slowly 13 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanol while cooling. The whole is stirred overnight at room temperature. The reaction mixture is evaporated and the residue is stirred with water. The oily product solidifies upon scratching. It is filtered off and crystallized from 4-methyl-2-pentanone, yielding 4 parts of cis - 1 - (2 - chloroethyl) - 4 - {4 - [2 - (2,4 - dichlorophenyl) - 2 - (1 H - imidazol - 1 - ylmethyl) - 1,3 - dioxolan - 4 - ylmethoxy]phenyl}piperazine; mp. 130.6°C.

A mixture of 2 parts of 1 H - isoindole - 1,3(2H) - dione, potassium salt, 6 parts of cis - 1 - (2 - chloroethyl) - 4 - {4 - [2 - (2,4 - dichlorophenyl) - 2 - (1H - imidazol - 1 - ylmethyl) - 1,3 - dioxolan - 4 - ylmethoxy]phenyl}piperazine and 100 parts of dimethyl sulfoxide is stirred and heated overnight at 60°C. The rection mixture is poured onto water and the whole is stirred for 10 minutes. The precipitated product is filtered off and dissolved in dichloromethane. The latter is dried, filtered and evaporated. The oily residue is purified by column-chromatography over silica gel using a mixture of trichloromethane and methanol (95:5 by volume) as eluent. The pure fractions are collected and the eluent is evaporated. The oily residue is crystallized from 2-propanol, yielding 4 parts of cis - 2 - {2 - [4 - {4 - [2 - (2,4 - dichlorophenyl) - 2 - (1H - imidazol - 1 - ylmethyl) - 1,3 - dioxolan - 4 - ylmethoxy]phenyl} - 1 - piperazinyl]ethyl} - 1 H - isoindole - 1,3(2H) - dione; mp. 169.1°C.

### B. Preparation of final compounds

### Example IV

To a stirred mixture of 4.1 parts of cis - [2 - (2,4 - dichlorophenyl) - 2 - (1 H - 1,2,4 - triazol - 1 - ylmethyl) - 1,3 - dioxolan - 4 - ylmethyl]methanesulfonate and 100 parts of dimethyl sulfoxide are added 0.25 parts of sodium hydride dispersion 76% and the whole is stirred and heated for 30 minutes at 50°C. After cooling, 4 parts of 1 - (4 - hydroxyphenyl) - 4 - (4 - methylphenylsulfonyl)-piperazine are added and the whole is stirred and heated overnight at 100°C. The reaction mixture is cooled and poured onto water. The product is extracted twice with benzene. The combined extracts are washed twice with a diluted sodium hydroxide solution and twice with water, dried, filtered and evaporated. The residue is converted into the hydrochloride salt in 2-propanol and 2,2'-oxybispropane. The salt is filtered off and crystallized from acetonitrile, yielding 2 parts of cis - 1 - {4 - [2 - (2,4 - dichlorophenyl) - 2 - (1 H - 1,2,4 - triazol - 1 - ylmethyl) - 1,3 - dioxolan - 4 - ylmethoxy]-phenyl} - 4 - (4 - methylphenylsulfonyl)piperazine dihydrochloride hemihydrate; mp. 187.5°C.

Following the same procedure and using equivalent amounts of 1 - (4 - hydroxyphenyl) - 4 - [(4 - morpholinyl)acetyl]piperazine and cis - [2 - (2,4 - dichlorophenyl) - 2 - (1 H - imidazol - 1 - ylmethyl) - 1,3 - dioxolan - 4 - ylmethyl]methanesulfonate there is also prepared: cis-1-{4-[2-(2,4-dichlorophenyl)-2-(1 H-imidazol-1-yl-methyl)-1,3-dioxolan-4-ylmethoxy]phenyl}-4-{2-(4-morpholinyl)acetyl]piperazine trinitrate; mp. 200°C.

### Example V

A mixture of 2.6 parts of 4-(acetylamino)benzenesulfonyl chloride, 4.9 parts of cis - 1 - {4- [2 - (2,4 - dichlorophenyl) - 2 - (1H - imidazol - 1 - ylmethyl) - 1,3 - dioxolan - 4 - ylmethoxy]-phenyl} - piperazine, 4 parts of potassium carbonate, 150 parts of trichloromethane and 100 parts of water is stirred for 2 hours at room temperature. The reaction mixture is diluted with water and the whole is stirred for another 2 hours at room temperature. The layers are separated and the organic phase is dried, filtered and evaporated. The residue is triturated in 4-methyl-2-pentanone. The product is filtered off and crystallized from 4-methyl-2-pentanone, yielding 6 parts (87%) of cis - N - {4 - [4 - {4 - [2 - (2,4 - dichlorophenyl) - 2 - (1H - imidazol - 1 - ylmethyl) - 1,3 - dioxolan - 4 - ylmethoxy] - phenyl} - 1 - piperazinylsulfonyl]phenyl}acetamide; mp. 205.8°C.

Following the same N-alkylafion-procedure and using equivalent amounts of the appropriate starting materials there are also prepared:
cis-1-{4-[2-(2,4-dichlorophenyl)-2-(1 H-imidazol-1-ylmethyl)-1,3-dioxolan-4-ylmethoxy]phenyl}-4-(phenylsulfonyl)piperazine ethanedioate (1:2); mp. 182.5°C.
cis-1-{4-[2-(2,4-dichlorophenyl)-2-(1 H-imidazol-1 -ylmethyl)-1,3-dioxolan-4-ylmethoxy]phenyl}-4-(4-methylphenylsulfonyl)piperazine; mp. 123.9°C;
cis-1-{4-[2-(2,4-dichlorophenyl)-2-(1 H-1,2,4-triazol-1-ylmethyl)-1,3-dioxolan-4-ylmethoxy]phenyl}-4-(phenylsulfonyl)piperazine; mp. 140.6°C; and
cis-N-{4-[4-{-[2-(2,4-dichlorophenyl)-2-( 1H-1,2,4-triazol-1-ylmethyl)-1,3-dioxolan-4-ylmethoxy]phenyl}-1-piperazinylsulfonyl]phenyl}acetamide; mp. 183.5°C.

### Example VI

A mixture of 1.8 parts of 2,2-dichloroacetyl chloride, 4.9 parts of cis - 1 - {4 - [2 - (2,4 - dichlorophenyl) - 2 - (1H - imidazol - 1 - ylmethyl) - 1,3 - dioxolan - 4 - ylmethoxy]phenyl}- piperazine, 2 parts of potassium carbonate and 150 parts of trichloromethane is stirred overnight at room temperature. Then there are added 50 parts of water and the layers are separated. The organic phase is dried, filtered and evaporated. The residue is purified by column-chromatography over silica gel using a mixture of trichloromethane and methanol (97:3 by volume) as eluent. The pure fractions are collected and the eluent is evaporated. The residue is converted into the ethanedioate salt in 2-propanone and 2,2'-oxybispropane. The salt is filtered off and crystallized from acetonitrile, yielding 3.6 parts (45%) of cis - 1 - (dichloroacetyl) - 4 - {4 - [2 - (2,4 - dichlorophenyl) - 2 - (1 H - imidazol - 1 - ylmethyl) - 1,3 - dioxolan - 4 - ylmethoxy]phenyl}piperazine ethanedioate (1:2) monohydrate; mp 98.9°C.

In a similar manner there are also prepared:
cis+trans-1-[3-(4-chlorophenyl)-1-oxo-2-propenyl]-4-{4-[2-(2,4-dichlorophenyl)-2-(1 H-1,2,4-triazol-1-ylmethyl)-1,3-dioxolan-4-ylmethoxy]phenyl}piperazine; mp. 180.1°C; and
cis-1-(dichloroacetyl)-4-{4-[2-(2,4-dichlorophenyl)-2-(1 H-1,2,4-triazol-1-ylmethyl)-1,3-dioxolan-4-ylmethoxy]phenyl}piperazine; mp. 142.4°C.

### Example VII

A mixture of 4.9 parts of trifluoromethanesulfonyl chloride, 2.1 parts of cis - 1 - {4 - [2 - (2,4 - dichlorophenyl) - 2 - (1H - 1,2,4 - triazol - 1 - ylmethyl) - 1,3 - dioxolan - 4 - ylmethoxy]-phenyl}piperazine and 100 parts of pyridine is stirred overnight at room temperature. The reaction mixture is poured onto water and the product is extracted with benzene. The extract is washed with water, dried, filtered and evaporated. The residue is purified by column-chromatography over silica gel using a mixture of methylbenzene and ethanol (95:5 by volume) as eluent. The pure fractions are collected and the eluent is evaporated. The residue is stirred in 2,2'-oxybispropane. The product is filtered off and dried, yielding 1.2 parts of cis - 1 - {4 - [2 - (2,4 - dichlorophenyl) - 2 - (1 H - 1,2,4 - triazol - 1 - ylmethyl) - 1,3 - dioxolan - 4 - ylmethoxy]phenyl} - 4 - (trifluoromethylsulfonyl)piperazine; mp. 166.9°C.

### Example VIII

A mixture of 1.34 parts of ethyl 2-chloroacetate, 5 parts of cis - 1 - {4 - [2 - (2,4 - dichlorophenyl) - 2 - (1H - imidazol - 1 - ylmethyl) - 1,3 - dioxolan - 4 - ylmethoxy]phenyl}piperazine, 2.75 parts of potassium carbonate and 50 parts of dimethyl sulfoxide is stirred and heated for 1 hour at 60°C. The reaction mixture is poured onto ice-water and the product is extracted with dichloromethane. The extract is washed with water, dried, filtered and evaporated. The oily residue is purified by column-chromatography over silica gel using a mixture of methylbenzene and ethanol (90:10 by volume) as eluent. The pure fractions are collected and the eluent is evaporated. The oily residue is crystallized from 4-methyl-2-pentanone. The product is filtered off and dried, yielding 0.6 parts of cis - ethyl - [4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazinylcarbonyloxy]acetate; mp. 146.6°C.

### Example IX

A mixture of 2.38 parts of 2 - chloro - N - (4 - nitrophenyl)acetamide, 4.8 parts of cis - 1 - {4 - [2 - (2,4 - dichlorophenyl) - 2 - (1H - imidazol - 1 - ylmethyl) - 1,3 - dioxolan - 4 - ylmethoxy]phenyl}piperazine, 1.2 parts of N,N-diethylethanamine and 68 parts of N,N-dimethylformamide is stirred for 4.50 hours at 80°C. The reaction mixture is poured onto 400 parts of water and the product is extracted three times with dichloromethane. The combined extracts are washed three times with water, dried, filtered and evaporated. The residue is purified by column-chromatography over silica gel using a mixture of trichloromethane and methanol (98:2 by volume) as eluent. The pure fractions are collected and the eluent is evaporated. The residue solidifies on scratching in 2,2'-oxybispropane. The product is filtered off and dried, yielding 5.6 parts of cis - 4 - {4 - [2 - (2,4 - dichlorophenyl) - 2 - (1 H - imidazol - 1 - ylmethyl) - 1,3 - dioxolan - 4 - ylmethoxy]phenyl} - N - (4 - nitrophenyl) - 1 - piperazineacetamide; mp. 151.1°C.

### Example X

Following the same procedure as described in Example IX and using equivalent amounts of the appropriate starting materials there are also prepared:
cis-4-{4-[2-(2,4-dichlorophenyl)-2-(1 H-imidazol-1-ylmethyl)-1,3-dioxolan-4-ylmethoxy]phenyl}-N-(2,6-dimethylphenyl)-1-piperazineacetamide trihydrochloride monohydrate; mp. 209.3°C.
cis-N-(2-chlorophenyl)-4-{4-[2-(2,4-dichlorophenyl)-2-(1 H-imidazol-1-ylmethyl)-1,3-dioxolan-4-ylmethoxy]phenyl}-1-piperazineacetamide; mp. 124.7°C;
cis-4-{4-[2-(2,4-dichlorophenyl)-2-(1 H-imidazol-1-ylmethyl)-1,3-dioxolan-4-ylmethoxy]phenyl}-N-(2-methylphenyl)-1-piperazineacetamide; mp. 123.8°C;
cis-4-{4-[2-12,4-dichlorophenyl)-2-(1 H-imidazol-1-ylmethyl)-1,3-dioxolan-4-ylmethoxy]phenyl}-N-(4-methylphenyl)-1-piperazineacetamide; mp. 124.1 °C;
cis-4-{4-[2-(2,4-dichlorophenyl)-2-(1 H-imidazol-1-ylmethyl)-1,3-dioxolan-4-ylmethoxy]phenyl}-N-(2-methoxyphenyl)-1-piperazineacetamide; mp. 153.5°C;
cis-4-{4-[2-(2,4-dichlorophenyl)-2-(1 H-imidazol-1-ylmethyl)-1,3-dioxolan-4-ylmethoxy]phenyl}-N-(4-methoxyphenyl)-1-piperazineacetamide; mp. 99.3°C;
cis-N-(2,6-dichlorophenyl)-4-{4-[2-(2,4-dichlorophenyl)-2-(1 H-imidazol-1-ylmethyl)-1,3-dioxolan-4-ylmethoxy]phenyl}-1-piperazineacetamide; mp. 127.2°C;
cis-N-(4-chlorophenyl)-4-{4-[2-(2,4-dichlorophenyl)-2-(1 H-imidazol-1-ylmethyl)-1,3-dioxolan-4-ylmethoxy]phenyl}-1-piperazineacetamide; mp. 138.1°C;
cis-N-(2,4-dichlorophenyl)-4-{4-[2-(2,4-dichlorophenyl)-2-(1 H-imidazol-1-ylmethyl)-1,3-dioxolan-4-ylmethoxy]phenyl}-1-piperazineacetamide; mp. 105.5°C;
cis-4-{4-[2-(2,4-dichlorophenyl)-2-(1 H-imidazol-1-ylmethyl)-1,3-dioxolan-4-ylmethoxy]phenyl}-N,N-dimethyl-1-piperazineacetamide; mp. 158.5°C;
cis-4-{4-[2-(2,4-dichlorophenyl)-2-(1 H-imidazol-1-ylmethyl)-1,3-dioxolan-4-ylmethoxy]phenyl}-N,N-diethyl-1-piperazineacetamide; mp. 120°C and
cis-4-{4-[2-(2,4-dichlorophenyl)-2-(1 H-imidazol-1-ylmethyl)-1,3-dioxolan-4-ylmethoxy]phenyl}-N,N-dipropyl-1-piperazineacetamide ethanedioate (1:4); mp. 113.9°C.

### Example XI

A mixture of 16 parts of chloroacetonitrile, 100 parts of cis - 1 - {4 - [2 - (2,4 - dichlorophenyl) - 2 - (1H - imidazol - 1 - ylmethyl) - 1,3 - dioxolan - 4 - ylmethoxy]phenyl}piperazine, 55.5 parts of N,N-diethylethanamine and 450 parts of N,N-dimethylformamide is stirred for 2 hours at 50°C. The reaction mixture is evaporated and the residue is stirred in water till the product is precipitated. It is filtered off and dissolved in dichloromethane. The organic phase is washed with water dried, filtered and evaporated. The residue is purified by column-chromatography over silica gel using a mixture of trichloromethane and methanol (97:3 by volume) as eluent. The pure fractions are collected and the eluent is evaporated. The residue is crystallized from 4-methyl-2-pentanone, yielding 65 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineacetonitrile hemihydrate; mp. 124.8°C.

Following the same procedure and replacing chloroacetonitrile by 1-(2-chloroethyl)piperidine hydrochloride respectively N-(2-bromoethyl)benzenamine hydrobromide, there are also prepared:
cis-1-{4-[2-(2,4-dichlorophenyl)-2-(1 H-imidazol-1-ylmethyl)-1,3-dioxolan-4-ylmethoxy]phenyl}-4-[2-(1-piperidinyl)ethyl]piperazine ethanedioate (1:3) dihydrate; mp. 133.6°C;
cis-4-{4-[2-(2,4-dichlorophenyl)-2-(1H-imidazol-1-ylmethyl)-1,3-dioxolan-4-ylmethoxy]phenyl}-N-phenyl-1-piperazineethanamine ethanedioate (1:2); mp. 188.6°C.

### Example XII

A mixture of 32 parts of cis - 2 - {2 - [4 - [2 - (2,4 - dichlorophenyl) - 2 - (1 H - imidazol - 1 - ylmethyl) - 1,3 - dioxolan - 4 - ylmethoxy]phenyl} - 1 - piperazinyl]ethyl} - 1 H - isoindole - 1,3(2H) - dione, 4 parts of sodium hydroxide and 80 parts of 1-butanol is stirred and refluxed overnight. Another 4 parts of sodium hydroxide and 10 parts of water are added and stirring is continued for 6 hours at reflux. The reaction mixture is evaporated and a lot of water is added to the residue. Evaporation is continued and the product is extracted with dichloromethane. The extract is washed with water, dried, filtered and evaporated. The oily residue is converted into the hydrochloride salt in 2-propanone. The salt is filtered off and crystallized from a mixture of 2-propanol and ethanol, yielding 14.5 parts of cis - 4 - {4 - [2 - (2,4 - dichlorophenyl) - 2 - (1 H - imidazol - 1 - ylmethyl) - 1,3 - dioxolan - 4 - ylmethoxy]-phenyl} - 1 - piperazineethanamine tetrahydrochloride.

A mixture of 10 parts of cis - 4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazineacetonitrile and 200 parts of methanol saturated with ammonia is hydrogenated at normal pressure and at room temperature with 2 parts of rhodium-on-charcoal catalyst 5%. After the calculated amount of hydrogen is taken up, the catalyst is filtered off and the filtrate is evaporated. The oily residue is purified by column-chromatography over silica gel using a mixture of trichloromethane and methanol (90:10 by volume) saturated with ammonia, as eluent. The pure fractions are collected and the eluent is evaporated. The residue is crystallized from 1,1'-oxybispropane, yielding 2 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine monohydrate; mp. 104.2°C.

### Example XIII

To a stirred mixture of 2.7 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine, 5 parts of N,N-diethylethanamine and 65 parts of dichloromethane is added a solution of 0.8 parts of dimethylcarbamic chloride in a small amount of dichloromethane. Stirring at room temperature is continued for 6 hours. The reaction mixture is evaporated. The oily residue is dissolved in 2-propanone. The solution is filtered and the filtrate is evaporated. The oily residue is purified by column-chromatography over silica gel using a mixture of trichloromethane and methanol (95:5 by volume) as eluent. The pure fractions are collected and the eluent is evaporated. The oily residue is converted into the ethanedioate salt in 2-propanone. The salt is filtered off and crystallized twice from ethanol, yielding 2.3 parts of cis - N - {2 - [4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazinyl]ethyl} - N',N' - dimethylurea ethanedioate (1:2) dihydrate; mp. 145.3°C.

In a similar manner there are also prepared:
cis-N-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}-N',N'-diethylurea ethanedioate (1:2) monohydrate; mp. 134.3°C;
cis-N-{2-[4-{4-[2-(2,4-dichlorophenyl)-2-( 1H-imidazol-1-ylmethyl)-1,3-dioxolan-4-ylmethoxy]phenyl}-1-piperazinyl]ethyl}-1-pyrrolidinecarboxamide ethanedioate (2:5) monohydrate; mp. 161.7°C; and
cis-N-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}-4-morpholinecarboxamide ethanedioate (1:2) monohydrate; mp. 155.8°C.

### Example XIV

A mixture of 2.8 parts of isocyanatomethane, 5 parts of cis - 4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazineethanol and 90 parts of methylbenzene is stirred and refluxed overnight. The reaction mixture is evaporated. The oily residue is purified by column-chromatography over silica gel using a mixture of trichloromethane and methanol (93:7 by volume) as eluent. The pure fractions are collected and the eluent is evaporated. The oily residue is crystallized from 2,2'-oxybispropane. The product is filtered off and dried, yielding 4 parts of cis - {2 - [4 - {4 - [2 - (2,4 - dichlorophenyl) - 2 - (1H - imidazol - 1 - ylmethyl) - 1,3 - dioxolan -4 - ylmethoxy]phenyl} - 1 - piperazinyl]ethyl}methylcarbamate; mp. 139.1°C.

Following the same procedure and using equivalent amounts of the appropriate starting materials there are also prepared:
cis-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}ethylcarbamate ethanedioate (1:2) monohydrate; mp. 170.7°C;
cis-{2-[4-{4-[2-(2,4-dichlorophenyl)-2-( 1H-imidazol-1-ylmethyl)-1,3-dioxolan-4-ylmethoxy]phenyl}-1-piperazinyl]ethyl}propylcarbamate ethanedioate (2:5) mp. 187°C;
cis-{2-[4-{4-[2-(2,4-dichlorophenyl)-2-( 1H-imidazol-1-ylmethyl)-1,3-dioxolan-4-ylmethoxy]phenyl}-1-piperazinyl]ethyl}phenylcarbamate ethanedioate (1:2); mp. 178.5°C and
cis-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}(1-methylethyl)carbamate ethanedioate (1:2); mp. 161 °C.

### Example XV

A mixture of 2.7 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine and 65 parts of dichloromethane is stirred till all solid enters solution. Then there is added a solution of 0.39 parts of isocyanatoethane in a small amount of dichloromethane. The whole is stirred for 1 hour at room temperature. The reaction mixture is evaporated and the residue is converted into the ethanedioate salt in ethanol. Upon scratching, the salt solidifies. It is filtered off and crystallized from ethanol, yielding 2.5 parts of cis - N - {2 - [4 - {4 - [2 - (2,4 - dichlorophenyl) - 2 - (1H - imidazol - 1 - ylmethyl) - 1,3 - dioxolan - 4 - ylmethoxy]phenyl} - 1 - piperazinyl]ethyl} - N' - ethylurea ethanedioate (1 :2) hemihydrate; mp. 126.4°C.

### Example XVI

Following the same procedure as described in Example XV and using equivalent amounts of the appropriately substituted amines and the appropriate isocyanates of isothiocyanates there are also prepared.
cis-N-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}-N'-methylurea monohydrate; mp. 102.7°C;
cis-N-(2-[4-{4-[2-(2,4-dichlorophenyl)-2-(1 H-imidazol-1-ylmethyl)-1,3-dioxolan-4-ylmethoxy]phenyl}-1-piperazinyl]ethyl}-N'-propylurea ethanedioate (1:2) monohydrate; mp. 125.3°C;
cis-N-butyl-N'-{2-[4-{4-[2-(2,4-dichlorophenyl)-2-(1 H-imidazol-1-ylmethyl)-1,3-dioxolan-4-ylmethoxy]phenyl}-1-piperazinyl]ethyl} urea ethanedioate (1:2) monohydrate; mp. 103.3°C;
cis-N-{2-[4-(4-[2-(2,4-dichlorophenyl)-2-( 1H-imidazol-1-ylmethyl)-1,3-dioxolan-4-ylmethoxy]phenyl}-1-piperazinyl]ethyl}-N'-phenylurea ethanedioate (1:2); mp. 183.9°C;
cis-N-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}-N'-(4-methylphenyl)urea ethanedioate (1:2) monohydrate; mp. 167°C;
cis-N-{2-[4-{4-[2-(2,4-dichlorophenyl)-2-(1 H-imidazol-1 -ylmethyl)-1,3-dioxolan-4-ylmethoxy]phenyl}-1-piperazinyl]ethyl}-N'-(2,6-dimethylphenyl)urea ethanedioate (1:2) monohydrate; mp. 142°C;
cis-N-{2-[4-{4-[2-(2,4-dichlorophenyl)-2-( 1H-imidazol-1-ylmethyl)-1,3-dioxolan-4-ylmethoxy]phenyl}-1-piperazinyl]ethyl}-N'-(4-methoxyphenyl)urea ethanedioate (1:2); mp. 172.8°C;
cis-N-(4-chlorophenyl)-N'-{2-[4-(4-[2-(2,4-dichlorophenyl)-2-(1 H-imidazoi-1-yimethyi)-1,3-dioxolan-4-ylmethoxy]phenyl}-1-piperazinyl]ethyl}urea ethanedioate (1:1) monohydrate; mp. 189.6°C;
cis-N-(2,6-dichlorophenyl)-N'-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}urea ethanedioate (1:2); mp. 170.1°C;
cis-N-(2,4-dichlorophenyl)-N'-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}urea ethanedioate (1:1) dihydrate; mp. 157.2°C;
cis-N-{2-[4-{4-[2-(2,4-dichlorophenyl)-2-(1 H-imidazol-1-y)methyl)-1,3-dioxolan-4-ylmethoxy]phenyl}-1-piperazinyl]ethyl}-N'-methylthiourea; mp. 163.7°C;
cis-N-{2-[4-{4-[2-(2,4-dichlorophenyl)-2-(1 H-imidazoi-1-ylmethyl)-1,3-dioxoian-4-ylmethoxy]phenyl}-1-piperazinyl]ethyl}-N'-ethylthiourea ethanedioate (1:2); mp. 172.3°C;
cis-N-{2-[4-{4-[2-(2,4-dichlorophenyl)-2-(1 H-imidazoi-1-ylmethyl)-1,3-dioxolan-4-ylmethoxy]phenyl}-1-piperazinyl]ethyl}-N'-(2-methylpropyl)thiourea ethane dioate (1:2); mp. 146.6°C;
cis-N-{2-[4-{4-[2-(2,4-dichlorophenyl)-2-(1 H-imidazot-1-ylmethyl)-1,3-dioxoian-4-ylmethoxy]phenyl}-1-piperazinyl]ethyl}-N'-phenylthiourea ethanedioate (1:2); mp. 185.2°C;
cis-N-(2-chlorophenyl)-N'-{2-[4-{4-[2-(2,4-dichlorophenyl)-2-(1 H-imidazol-1-ylmethyl)-1,3-dioxolan-4-ylmethoxy]phenyl}-1-piperazinyl]ethyl}thiourea ethanedioate (1:2); mp. 176°C;
cis-N-(4-chlorophenyl)-N'-{2-[4-{4-[2-(2,4-dichlorophenyl)-2-( 1H-imidazol-1-ylmethyl)-1,3-dioxolan-4-ylmethoxy]phenyl}-1-piperazinyl]ethyl}thiourea ethanedioate (1:2); mp. 214.7°C;
cis-N-(2,4-dichlorophenyl)-N'-{2-[4-{4-[2-(2,4-d ich lorophenyl)-2-( 1H-imidazol-1-ylmethyl)-1,3-dioxolan-4-ylmethoxy]phenyl}-1-piperazinyl]ethyl}thiourea ethanedioate (1:2); mp. 171°C; and
cis-N-{2-[4-{4-[2-(2,4-dichlorophenyl)-2-(1 H-imidazoi-1-ylmethyl)-1,3-dioxolan-4-

ylmethoxy]phenyl}-1-piperazinyl]ethyl}-N'-(4-methoxyphenyl)thiourea ethanedioate (1:2); mp. 197.8°C.

### Example XVII

A mixture of 60 parts of formic acid, 5 parts of cis - 4 - {4 - [2 - (2,4 - dichlorophenyl) - 2 - (1H - imidazol - 1 - ylmethyl) - 1,3 -dioxolan -4 -ylmethoxy]phenyl} - 1 -piperazineethanol and 5 parts of acetic acid anhydride is stirred and refluxed for 2 hours. The reaction mixture is evaporated and the residue is taken up in water. Sodium hydrogen carbonate is added and the whole is stirred for 10 minutes at room temperature. The product is extracted with dichloromethane. The extract is dried, filtered and evaporated. The oily residue is crystallized from a mixture of benzene and petroleumether. The product is filtered off and recrystallized from 4-methyl-2-pentanone, yielding 2.5 parts of cis - {2-[4 - [2 - (2,4 - dichlorophenyl) - 2 - (1H - imidazol - 1 - ylmethyl) - 1,3 - dioxolan - 4 - ylmethoxy]phenyl} - 1 - piperazinyl]ethyl} formate; mp. 109.1°C.

### Example XVIII

A mixture of 12 parts of formic acid and 2.7 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine is stirred and refluxed overnight. The reaction mixture is evaporated. The oily residue is dissolved in water and the solution is neutralized with sodium hydrogen carbonate. The product is extracted with trichloromethane. The extract is dried, filtered and evaporated. The residue is purified by column-chromatography over silica gel using a mixture of trichloromethane and methanol (98:2 by volume) saturated with ammonium hydroxide, as eluent. The pure fractions are collected and the eluent is evaporated. The residue is crystallized from 4-methyl-2-pentanone, yielding 0.7 parts of cis - N - {2 - [4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazinyl]ethyl} formamide; mp. 152.4°C.

### Example XIX

A mixture of 10 parts of acetic acid anhydride, 5 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazine ethanol and 130 parts of dichloromethane is stirred and refluxed for 1 hour. The reaction mixture is evaporated. The oily residue solidifies on scratching in 2,2'-oxybispropane. The product is filtered off and crystallized from a mixture of benzene and petroleumether, yielding 3.6 parts of cis - {2 - [4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazinyl]ethyl}acetate; mp. 174.7°C.

In a similar manner there are also prepared:
cis-{2-[4-{4-[2-12,4-dich lorophenyl)-2-( 1H-imidazol-1-ylmethyl)-1,3-dioxolan-4-ylmethoxy]phenyl}-1-piperazinyl]ethyl}propanoate; mp. 145.9°C; and
cis-{2-[4-{4-[2-(2,4-dichlorophenyl)-2-(1 H-imidazoi-1-ylmethyl)-1,3-dioxotan-4-ylmethoxy]phenyl}-1-piperazinyl]ethyl}butanoate; mp. 96.6°C.

### Example XX

To a stirred mixture of 2.7 parts of cis - N - 4-{4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine and 130 parts of dichloromethane is added 1 part of acetic acid anhydride and stirring is continued overnight at room temperature. The reaction mixture is washed with a sodium hydrogen carbonate solution, dried, filtered and evaporated. The oily residue is converted into the ethanedioate salt in 2-propanone. The salt is filtered off and crystallized from a mixture of acetonitrile and ethanol, yielding 3 parts of cis - N - {2 - [4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazinyl]ethyl}acetamide ethanedioate (1:2) monohydrate; mp. 139.1°C.

### Example XXI

A mixture of 3.5 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine and 65 parts of dichloromethane is stirred till all solid enters solution. Then there are added 1 part of sodium hydrogen carbonate and 50 parts of water. While stirring vigorously, 0.7 parts of methyl carbonochloridate are added and the whole is stirred for 3 hours at room temperature. The organic phase is separated, washed with water, dried, filtered and evaporated. The oily residue is purified by column-chromatography over silica gel using a mixture of trichloromethane and methanol (95:5 by volume) as eluent. The pure fractions are collected and the eluent is evaporated. The residue is converted into the ethanedioate salt in methanol. The salt is filtered off and dried, yielding 3.5 parts of cis - methyl {2 - [4 - {4 - [2 - (2,4 - dichlorophenyl) - 2 - (1H - imidazol - 1 - ylmethyl) - 1,3 - dioxolan - 4 - ylmethoxy]phenyl} - 1 - piperazinyl]ethyl} carbamate ethanedioate (1:2) dihydrate; mp. 168.9°C.

In a similar manner there are also prepared:
cis-ethyl {2-(4-{4-[2-(2,4-dichlorophenyl)-2-(1H-imidazol-1-ylmethyl)-1,3-dioxolan-4-ylmethoxy]phenyl}-1-piperazinyl]ethyl} carbamate ethanedioate (1 :2) monohydrate; mp. 164.1 °C; and
cis-phenyl {2-[4-{4-[2-(2,4-dichlorophenyl)-2-(1H-imidazol-1-ylmethyl)-1,3-dioxolan-4-ylmethoxy]phenyl}-1-piperazinyl]ethyl} carbamate ethanedioate (1:2); mp. 179.6°C.

### Example XXII

A mixture of 5 parts of cis - 4 - {4 - [2 - (2,4 - dichlorophenyl) - 2 - (1 H - imidazol - 1 - ylmethyl) - 1,3 - dioxolan - 4 - ylmethoxy]-phenyl} - 1 - piperazineethanol, 6 parts of N,N-diethyl- ethanamine and 130 parts of dichloromethane is stirred till all solid enters solution. Then there are added 2.7 parts of 2-methylpropanoyl chloride and the whole is stirred and refluxed for 2 hours. The reaction mixture is cooled and washed with water. The organic phase is dried, filtered and evaporated. The oily residue is purified by column-chromatography over silica gel using a mixture of trichloromethane and methanol (95:5 by volume) as eluent. The pure fractions are collected and the eluent is evaporated. The residue is converted into the ethanedioate salt in ethanol and 1,1'-oxybisethane. The salt is filtered off and crystallized from ethanol, yielding 3.5 parts of cis - {2 - [4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazinyl]ethyl} - 2 - methylpropanoate ethanedioate (1:2) monohydrate; mp. 156.5°C.

### Example XXIII

A mixture of 2 parts of 1 - chloro - 3 - (chloromethyl)benzene, 4.9 parts of cis - 1 - {4 - [2 - (2,4 - dichlorophenyl) - 2 - (1 H - imidazol - 1 - ylmethyl) - 1,3 - dioxolan - 4 - ylmethoxy]-phenyl}piperazine, 1.2 parts of N,N-diethylethanamine and 68 parts of N,N-dimethylformamide is stirred and heated for 3.50 hours at 80°C. The whole is further stirred over week-end at room temperature. The reaction mixture is poured onto water and the product is extracted three times with benzene. The combined extracts are washed three times with water, dried, filtered and evaporated. The residue is purified by column-chromatography over silica gel using a mixture of trichloromethane and methanol (98:2 by volume) as eluent. The pure fractions are collected and the eluent is evaporated. The residue is triturated in 2,2'-oxybispropane, yielding 3.4 parts of cis - 1 - (3 - chlorophenylmethyl) - . 4 - {4 - [2 - (2,4 - dichlorophenyl) - 2 - (1H - imidazol - 1 - ylmethyl) - 1,3 - dioxolan - 4 - y(methoxy]phenyl}piperazine; mp. 109.9°C.

### Example XXIV

There are also prepared following the same procedure as described in Example XXIII and using equivalent amounts of the appropriate starting materials:
cis-1-(2-chlorophenylmethyl)-4-{4-[2-(2,4-dichlorophenyl)-2-(1H-imidazol-1-ylmethyl)-1,3-dioxolan-4-ylmethoxy]phenyl}piperazinyl; mp. 108.8°C;
cis-1-(4-chlorophenylmethyl)-4-{4-[2-(2,4-dichlorophenyl)-2-(1H-imidazol-1-ylmethyl)-1,3-dioxolan-4-ylmethoxylphenyl}piperazinyl; mp. 126.3°C;
cis-1-{4-[2-(2,4-dichlorophenyl)-2-(1H-imidazol-1-ylmethyl)-1,3-dioxolan-4-ylmethoxy]phenyl}-4-(2,4-dichlorophenylmethyl)piperazine; mp. 112.4°C;
cis-1-{4-[2-(2,4-dichlorophenyl)-2-(1H-imidazol-1-ylmethyl)-1,3-dioxolan-4-ylmethoxy]phenyl}-4-(2,6-dichlorophenylmethyl)piperazine; mp. 134°C;
cis-1-(4-[2-(2,4-dichlorophenyl)-2-(1H-imidazol-1-ylmethyl)-1,3-dioxolan-4-ylmethoxy]phenyl}-4-(2,5-dimethylphenylmethyl)piperazine; mp. 100.3°C;
cis-1-{4-[2-(2,4-dichlorophenyl)-2-(1H-imidazol-1-ylmethyl)-1,3-dioxolan-4-vlmethoxy]phenyl}-4-[3-(trifluoromethyl)phenylmethyl]piperazine; ethanedioate (2:5) mp. 207.2°C;
cis-1-(2-chloro-4-nitrophenylmethyl)-4-{4-[2-(2,4-dichlorophenyl)-2-(1H-imidazol-1-ylmethyl)-1,3-dioxolan-4-ylmethoxy]phenyl}piperazine; mp. 124.5°C;
cis-1-{4-[2-(2,4-dichlorophenyl)-2-1(H-imidazol-1-ylmethyl)-1,3-dioxolan-4-ylmethoxy]phenyl}-4-(4-nitrophenylmethyl)piperazine; mp. 160.5°C;
cis-1-[2-(4-chlorophenyl)ethyl]-4-{4-[2,4-dichlorophenyl)-2-(1H-imidazol-1-ylmethyl)-1,3-dioxolan-4-ylmethoxy]phenyl}piperazine; mp. 106―108°C;
cis-1-(4-bromophenylmethyl)-4-{4-[2-(2,4-dichlorophenyl)-2-(1H-imidazol-1-ylmethyl)-1,3-dioxolan-4-ylmethoxy]phenyl}piperazine hemihydrate; mp. 118.8°C;
cis-1-{4-[2-(2,4-dichlorophenyl)-2-(1H-imidazol-1-ylmethyl)-1,3-dioxolan-4-ylmethoxy]phenyl}-4-(4-fluorophenylmethyl)piperazine; mp. 137.9°C;
cis-1-{4-[2-(2,4-dichlorophenyl)-2-(1H-imidazol-1-ylmethyl)-1,3-dioxolan-4-ylmethoxy]phenyl}-4-(4-methylphenylmethyl)piperazine; mp. 109.8°C;
cis-1-{4-[2-(2,4-dichlorophenyl)-2-(1H-imidazol-1-ylmethyl)-1,3-dioxolan-4-ylmethoxy]phenyl}-4-(4-methoxyphenylmethyl)piperazine; mp. 113.3°C;
cis-1-{4-[2-(2,4-dichlorophenyl)-2-(1H-imidazol-1-ylmethyl)-1,3-dioxolan-4-ylmethoxy]phenyl}-4-[2-(4-methoxyphenyl)ethyl]piperazine; mp. 137.9°C;
cis-1-(4-chlorophenylmethyl)-4-{4-[2-(2,4-dichlorophenyl)-2-(1H-1,2,4-triazol-1-ylmethyl)-1,3-dioxolan-4-ylmethoxy]phenyl}piperazine; mp. 125.1°C;
cis-1-(2-chlorophenylmethyl)-4-{4-[2-(2,4-dichlorophenyl)-2-(1H-1,2,4-triazol-1-ylmethyl)-1,3-dioxolan-4-ylmethoxy]phenyl}piperazine; mp. 92.5°C;
cis-1-[2-(4-chlorophenyl)ethyl]-4-{4-[2-(2,4-dichlorophenyl)-2-(1H-1,2,4-triazol-1-ylmethyl)-1,3-dioxolan-4-ylmethoxy]phenyl}piperazine; mp. 120°C;
cis-1-(4-bromophenylmethyl)-4-{4-[2-(2,4-dichlorophenyl)-2-(1H-1,2,4-triazol-1-ylmethyl)-1,3-dioxolan-4-ylmethoxy]phenyl}piperazine; mp. 139.2°C;
cis-1-{4-[2-(2,4-dichlorophenyl)-2-(1H-1,2,4-triazol-1-ylmethyl)-1,3-dioxolan-4-ylmethoxy]-phenyl}-4-(4-fluorophenylmethyl)piperazine; mp. 111.5°C;
cis-1-(2,4-dichlorophenylmethyl)-4-{4-[2-(2,4-dichlorophenyl)-2-(1H-1,2,4-triazol-1-ylmethyl)-1,3-dioxolan-4-ylmethoxy]phenyl}piperazine; mp. 118.8°C;
cis-1-{4-[2-(2,4-dichlorophenyl)-2-(1H-1,2,4-triazol-1-ylmethyl)-1,3-dioxolan-4-ylmethoxy]-phenyl}-4-(4-methylphenylmethyl)piperazine; mp. 123.4°C;
cis-1-{4-[2-(2,4-dichlorophenyl)-2-(1H-1,2,4-triazol-1-ylmethyl)-1,3-dioxolan-4-ylmethoxy]-phenyl}-4-(2,5-dimethylphenylmethyl)piperazine; mp. 100.2°C;
cis-1-{4-[2-(2,4-dichlorophenyl)-2-(1H-1,2,4-triazol-1-ylmethyl)-1,3-dioxolan-4-ylmethoxy]-phenyl}-4-(4-methoxyphenylmethyl)piperazine; mp. 112°C;
cis-1-{4-[2-(2,4-dichlorophenyl)-2-(1H-1,2,4-triazol-1-ylmethyl)-1,3-dioxolan-4-ylmethoxy]-phenyl}-4-[2-(4-methoxyphenyl)ethyl]piperazine; mp. 139.9°C;
cis-1-{4-[2-(2,4-dichlorophenyl)-2-(1H-imidazol-1-ylmethyl)-1,3-dioxolan-4-ylmethoxy]phenyl}-4-(2-pyridinylmethyl)piperazine; mp. 111.3°C;
cis-1-{4-[2-(2,4-dichlorophenyl)-2-(1H-imidazol-1-ylmethyl)-1,3-dioxolan-4-ylmethoxy]phenyl}-4-(3-pyridinylmethyl)piperazine; mp. 100.9°C;
cis-1-{4-[2-(2,4-dichlorophenyl)-2-(1H-imidazol-1-ylmethyl)-1,3-dioxolan-4-ylmethoxy]phenyl}-4-(4-pyridinylmethyl)piperazine; mp. 110.6°C;
cis-1-{4-[2-(2,4-dichlorophenyl)-2-(1H-1,2,4-triazol-1-ylmethyl)-1,3-dioxolan-4-ylmethoxy]-phenyl}-4-(4-pyridinylmethyl)piperazine; mp. 98.2°C;
cis-1-{4-[2-(2,4-dichlorophenyl)-2-(1H-1,2,4-triazol-1-ylmethyl)-1,3-dioxolan-4-ylmethoxy]-phenyl}-4-(2-pyridinylmethyl)piperazine; mp. 104.4°C; and
cis-1-{4-[2-(2,4-dichlorophenyl)-2-(1H-1,2,4-triazol-1-ylmethyl)-1,3-dioxolan-4-ylmethoxy]-phenyl}-4-(3-pyridinylmethyl)piperazine; mp. 106.2°C;

### Example XXV

To 1 part of a solution of 2 parts of thiophene in 40 parts of ethanol are added 4 parts of 2-propanone, 2.7 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineethanamine and 120 parts of methanol. The whole is hydrogenated at normal pressure and at room temperature with 2 parts of platinum-on-charcoal catalyst 5%. After the calculated amount of hydrogen is taken up, the catalyst is filtered off and the filtrate is evaporated. The residue is converted into th ethanedioate salt in 2-propanone. The salt is filtered off and crystallized from a mixture of ethanol and 2-propanone, yielding 2.7 parts of cis - 4 - {4 - [2 - (2,4 - dichlorophenyl) - 2 - (1H - imidazol - 1 - ylmethyl) - 1,3 - dioxolan - 4 - ylmethoxy]phenyl} - N - (1 - methylethyl) - 1 - piperazineethanamine ethanedioate (1:3) dihydrate; mp. 128.3°C.

### Example XXVI

4.6 Parts of cis + trans - 1 - [3 - (4 - chlorophenyl) - 1 - oxo - 2 - propenyl] - 4 - {4 - [2 - (2,4 - dichlorophenyl) - 2 - (1H - 1,2,4 - triazol - 1 - ylmethyl) - 1,3 - dioxolan - 4 - ylmethoxy]-phenyl}piperazine are purified by column-chromatography over silica gel using a mixture of trichloromethane, hexane and methanol (48:48:4 by volume) as eluent. The fractions, containing the cis-isomer, are collected and the eluent is evaporated. The residue is crystallized from 4-methyl-2-pentanone. The product is filtered off and dried, yielding 1.9 parts of cis - 1 - [3 - (4 - chlorophenyl) - 1 - oxo - 2 - propenyl] - 4 - {4 - [2 - (2,4 - dichlorophenyl) - 2 - (1H - 1,2,4 - triazol - 1 - ylmethyl) - 1,3 - dioxolan - 4 - ylmethoxy]phenyl}piperazine; mp. 185°C.

### Example XXVII

A mixture of 6.8 parts of cis - N - {4 - [4 - 4 - [2 - (2,4 - dichlorophenyl) - 2 - (1H - imidazol - 1 - ylmethyl) - 1,3 - dioxolan - 4 - ylmethoxy]phenyl} - 1 - piperazinylsulfonyl]phenyl acetamide, 0.8 parts of sodium hydroxide and 80 parts of 1-butanol is stirred and refluxed overnight. Benzene is added and the whole is washed with water. The solvent is evaporated. The residue is triturated in a mixture of 4-methyl-2-pentanone and 2,2'-oxybispropane. The product is filtered off and crystallized from 4-methyl-2-pentanone, yielding 6.2 parts (94%) of cis - 1 - (4 - aminophenylsulfonyl) - 4 - {4 - [2 - (2,4 - dichlorophenyl) - 2 - (1H - imidazol - 1 - ylmethyl) - 1,3 - dioxolan - 4 - ylmethoxy]phenyl} - piperazine monohydrate; mp. 147.8°C.

## Claims (Claims for the following Contracting State(s) : s: BE CH DE FR GB IT LU NL SE)

1. A chemical compound selected from the group consisting of an azole derivative having the formula: 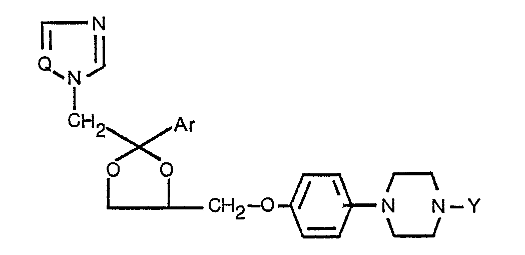 and the pharmaceutically acceptable acid addition salts and stereochemically isomeric forms thereof, wherein:
Q is a member selected from the group consisting of N and CH;
Ar is a member selected from the group consisting of phenyl, thienyl, halothienyl and substituted phenyl, said substituted phenyl having from 1 to 3 substituents each independently selected from the group consisting of halo, C₁―C₆ alkyl, C₁―C₆ alkyloxy and trifluoromethyl; and the radical Y is a member selected from the group consisting of:
a radical of the formula 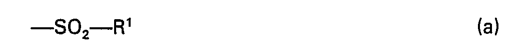 wherein R' is selected from the group consisting of trifluoromethyl and aryl;
a radical of formula 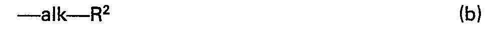 wherein alk is a member selected from the group consisting of C₁―C₆ alkylene and C₂―C₆ alkenylene and R² is a member selected from the group consisting of cyano, amino, mono- and di(C₁―C₆ alkyl)amino, arylamino, mono- and di(aryl C₁―C₆ alkyl)amino, 1-pyrrolidinyl, 1-morpholinyl, 1-piperidinyl, aryloxy and aryl, provided that alk is other than methylene when R² is phenyl;
a radical of formula 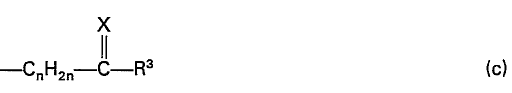 wherein n is an integer of from 0 to 6 inclusive, X is 0 or S and R³ is selected from the group consisting of hydrogen, mono-, di- and trihalo-C₁―C₆-alkyl, amino, mono- and di(C₁―C₆ alkyl)amino, arylamino, mono- and di(aryl C₁―C₆ alkyl)amino, amino C₁―C₆ alkyl, mono- and di(C₁―C₆ alkyl)amino C₁―C₆ alkyl, (1-pyrrolidinyl) C₁―C₆ alkyl, (1-morpholinyl) C₁―C₆ alkyl, (1-piperidinyl) C₁―C₆ alkyl, aryl, aryl C₁―C₆ alkyl, aryl C₂―C₆ alkenyl and C₁―C₆ alkyloxycarbonyl C₁―C₆alkyloxy, provided that:
i) said n is other than 0 or 1 when said R³ is amino or C₁―C₆ alkylamino; and
ii) said n is other than 0 when said R³ is hydrogen di(C₁―C₆ alkyl)amino or aryl; and
a radical of formula 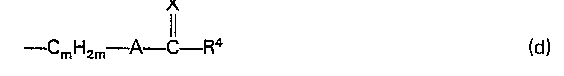 wherein m is an integer of from 1 to 6 inclusive, A is 0 or NH, X is 0 or S and R⁴ is a member selected from the group consisting of hydrogen, C₁―C₆ alkyl, C₁―C₆ alkyloxy, aryl, aryloxy, aryl C₁―C₆ alkyl, amino, mono- and di(C₁―C₆ alkyl)amino, arylamino, mono- and di(aryl C₁―C₆ alkyl)amino, 1-pyrrolidinyl, 1-morpholinyl and 1-piperidinyl;
wherein said aryl, as used in the foregoing definitions, is selected from the group consisting of phenyl, substituted phenyl, thienyl, halothienyl, C₁―C₆ alkylthienyl and pyridinyl, said substituted phenyl having from 1 to 3 substituents each independently selected from the group consisting of C₁―C₆ alkyl, C₁―C₆ alkyloxy, halo, amino, mono- and di(C₁―C₆ alkyl)amino, C₁―C₆ alkylcarbonylamino, nitro and trifluoromethyl.

2. A chemical compound according to claim 1 selected from the group consisting of cis - ethyl [4 - {4 - [2 - (2,4 - dichlorophenyl) - 2 - (1 H - imidazol - 1 - ylmethyl) - 1,3 - dioxolan - 4 - ylmethoxy] - phenyl} - 1 - piperazinylcarbonyloxy]acetate and the pharmaceutically acceptable acid addition salts and stereochemically isomeric forms thereof.

3. A chemical compound according to claim 1 selected from the group consisting of cis - 4 - {4 - [2 - (2,4 - dichlorophenyl) - 2 - (1 H - imidazol - 1 - ylmethyl) - 1,3 - dioxolan - 4 - ylmethoxy]phenyl} - N - (2 - methylphenyl) - 1 - piperazineacetamide and the pharmaceutically acceptable acid addition salts and stereochemically isomeric forms thereof.

4. A chemical compound according to claim 1 selected from the group consisting of cis - N - (2,6 - dichlorophenyl) - 4 - {4 - [2 - (2,4 - dichlorophenyl) - 2 - (1 H - imidazol - 1 - ylmethyl) - 1,3 - dioxolan - 4 - ylmethoxy]phenyl} - 1 - piperazineacetamide and the pharmaceutically acceptable acid addition salts and stereochemically isomeric forms thereof.

5. A composition for combatting a microorganism selected from the group consisting of fungus and bacterium comprising an inert carrier material and as an active ingredient an effective antifungal or antibacterial amount of a compound according to any one of claims 1 to 4.

6. A chemical compound according to claim 1 selected from the group consisting of cis - 1 - {4 - [2 - (2,4 - dichlorophenyl) 2 - (1 H - imidazol - 1 - ylmethyl) - 1,3 - dioxolan - 4 - ylmethoxy]phenyl} - 4 - (2 - pyridinylmethyl)piperazine and the pharmaceutically acceptable acid addition salts and stereochemically isomeric forms thereof.

7. A process for preparing a chemical compound according to claim 1, characterized by
a) reacting a compound of the formula 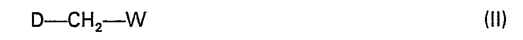 wherein D is 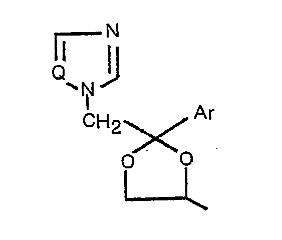 and Q is CH or N and W is a reactive ester residue with a compound of the formula 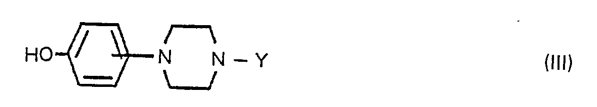 the reaction being carried out in an appropriate reaction-inert organic solvent at elevated temperatures and, if desired, first converting the substituted phenol (III) into a metal salt thereof, and to use thereafter said metal salt in the reaction with (II); or
b) reacting a compound of the formula 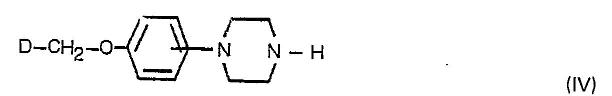 with a compound of the formula 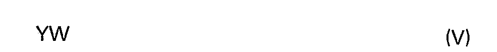 in said reaction the meaning of W as well as the reaction-conditions depending upon the nature of Y;
b) i) in case Y is other than a radical of formula (a), other than a radical of formula (c) wherein n is 0 and other than a radical of formula (c) wherein n is 1 and R³ is C₁―C₆ alkyloxycarbonyl C₁―C₆ alkyloxy, W may be halo, or a sulfonyloxy group and the reaction is then preferably conducted in a suitable reaction-inert solvent;
b) ii) in case Y represents a radical of formula (a) or a radical of formula (c) wherein n is 0, then W is selected from the group consisting of halo, most preferably chloro, and the reaction is preferably conducted in a suitable reaction-inert solvent; or
c) preparing compounds of formula (I) wherein Y represents a radical (b) wherein alk is as previously defined and wherein R² is as previously defined, but other than cyano and other than aryl, said R² being represented by R2 by substituting the reactive leaving group W in an intermediate of formula 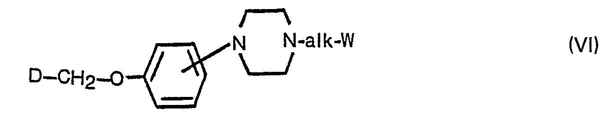 with a compound of the formula 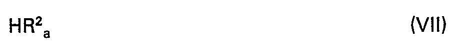 in order to prepare a compound of the formula 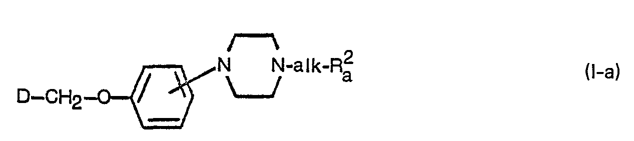 or
c) i) preparing compounds of formula (I-a) wherein R² is amino by reacting (VI) with ammonia wherein 2 protons have been replaced by protective groups, and subsequently eliminating the protective groups following art-known procedures; or
c) ii) preparing primary amine derivatives of formula (I-a) from the corresponding cyanides by reducing the latter following art-known cyano-to-amine reduction procedures such as, catalytic hydrogenation; or
c) iii) preparing compounds of formula (I-a) wherein R²ₐ is amino, mono- and di(C₁―C₆ alkyl)amino, arylamino, mono- and di(aryl C₁―C₆ alkyl)amino, 1-pyrrolidinyl, 1-morpholinyl or 1-piperidinyl starting from an appropriate carbonyl compound and ammonia or an appropriately substituted primary or secondary amine following art-known reductive amination procedures; or
d) preparing the compounds of formula (I) wherein Y represents a radical (d) wherein A and R⁴ are as previously defined and wherein X is 0, by reacting an amine or an alcohol of formula 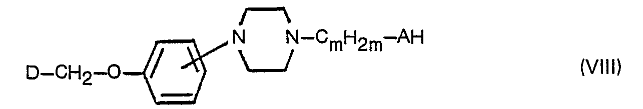 with a compound of the formula 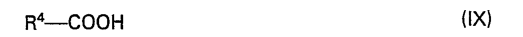 or a functional derivative thereof, such as its acyl halide 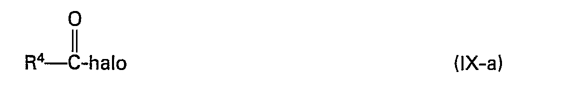 or its anhydride 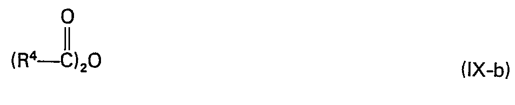 in order to prepare a compound of the formula 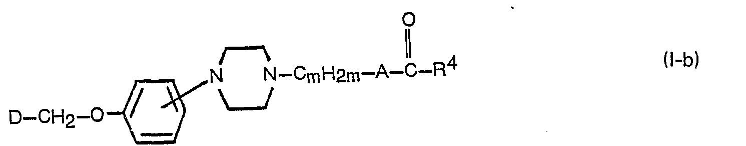 or
d) preparing compounds of formula (I) wherein Y represents a radical (e) wherein A and X are as previously defined and R⁴ is amino, C₁―C₆ alkylamino, arylamino or aryl C₁―C₆ alkylamino, said R⁴ being represented by -NHR4a by the addition-reaction of an appropriate amine or alcohol of formula (VIII) with an appropriately substituted isocyanate or isothiocyanate of formula 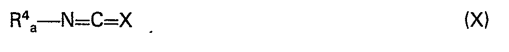 in order to prepare a compound of the formula 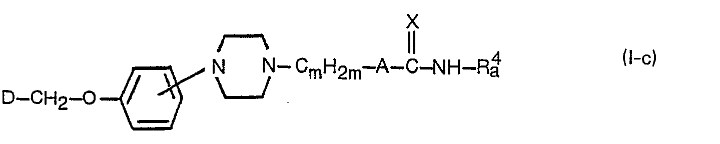 the addition-reaction being preferably carried out by stirring and heating the reactants together in the presence of a suitable reaction-inert solvent; and, if desired, preparing pharmaceutically acceptable acid addition salts or stereochemically isomeric forms of the products thereof.

8. A pharmaceutical composition comprising an inert carrier material and as an active ingredient an effective anti-fungal or antibacterial amount of a compound in accordance with Claim 6.

9. A compound in accordance with any one of Claims 1 to 4 or 6, or a composition in accordance with Claim 5 or Claim 8, for use as an anti-fungal and/or an antibacterial agent.

## Claims (Claims for the following Contracting State(s) : AT)

1. A composition for combatting a microorganism selected from the group consisting of fungus and bacterium comprising an inert carrier material and as an active ingredient an effective anti-fungal or antibacterial amount of a compound selected from the group consisting of an azole derivative having the formula: 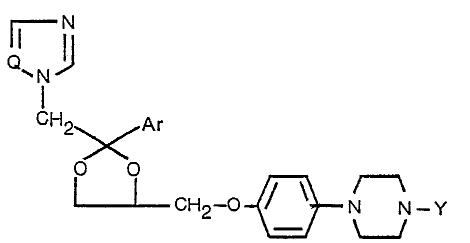 and the pharmaceutically acceptable acid addition salts and stereochemically isomeric forms thereof, wherein:
Q is a member selected from the group consisting of N and CH;
Ar is a member selected from the group consisting of phenyl, thienyl, halothienyl and substituted phenyl, said substituted phenyl having from 1 to 3 substituents each independently selected from the group consisting of halo, C₁―C₄ alkyl, C₁―C₄ alkyloxy and trifluoromethyl; and the radical Y is a member selected from the group consisting of:
a radical of the formula 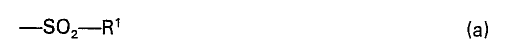 wherein R' is selected from the group consisting of trifluoromethyl and aryl;
a radical of formula 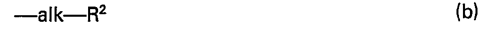 wherein alk is a member selected from the group consisting of C₁―C₆ alkylene and C₂―C₆ alkenylene and R² is a member selected from the group consisting of cyano, amino, mono- and di(C₁―C₆ alkyl)amino, arylamino, mono- and di(aryl C₁―C₆ alkyl)amino, 1-pyrrolidinyl, 1-morpholinyl, 1-piperidinyl, aryloxy and aryl, provided that alk is other than methylene when R² is phenyl;
a radical of formula 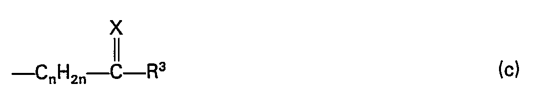 wherein n is an integer of from 0 to 6 inclusive, X is 0 or S and R³ is selected from the group consisting of hydrogen, mono-, di- and trihalo-C₁―C₆-alkyl, amino, mono- and di(C₁―C₆ alkyl)amino, arylamino, mono- and di(aryl C₁―C₆ alkyl)amino, amino C₁―C₆ alkyl, mono- and di(C₁―C₆ alkyl)amino C₁―C₆ alkyl, (1-pyrrolidinyl) C₁―C₆ alkyl, (1-morpholinyl) C₁―C₆ alkyl, (1-piperidinyl) C₁―C₆ alkyl, aryl, aryl C₁―C₆ alkyl, aryl C₂―C₆ alkenyl and C₁―C₆ alkyloxycarbonyl C₁―C₆alkyloxv. orovided that:
i) said n is other than 0 or 1 when said R³ is amino or C₁―C₆ alkylamino; and
ii) said n is other than 0 when said R³ is hydrogen di(C₁―C₆ alkyl)amino or aryl; and
a radical of formula 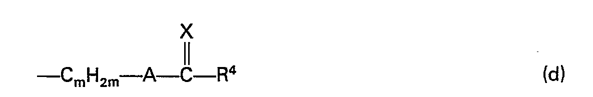 wherein m is an integer of from 1 to 6 inclusive, A is O or NH, X is O or S and R⁴ is a member selected from the group consisting of hydrogen, C₁―C₆ alkyl, C₁―C₆ alkyloxy, aryl, aryloxy, aryl C₁―C₆ alkyl, amino, mono- and di(C₁―C₆ alkyl)amino, arylamino, mono- and di(aryl C₁―C₆ alkyl)amino, 1-pyrrolidinyl, 1-morpholinyl and 1-piperidinyl;
wherein said aryl, as used in the foregoing definitions, is selected from the group consisting of phenyl,
substituted phenyl, thienyl, halothienyl, C₁―C₆ alkylthienyl and pyridinyl, said substituted phenyl having from 1 to 3 substituents each independently selected from the group consisting of C₁―C₆ alkyl, C₁―C₆ alkyloxy, halo, amino, mono- and di(C₁―C₆ alkyl)amino, C₁―C₆ alkylcarbonylamino, nitro and trifluoromethyl.

2. A composition according to Claim 1 wherein said chemical compound is selected from the group consisting of cis - 1 - {4 - [2 - (2,4 - dichlorophenyl) - 2 - (1 H - imidazol - 1 - ylmethyl) - 1,3 - dioxolan - 4 - ylmethoxy]phenyl} - 4 - (2 - pyridinylmethyl)piperazine and the pharmaceutically acceptable acid addition salts and stereochemically isomeric forms thereof.

3. A composition according to claim 1 wherein said chemical compound is selected from the group consisting of cis - ethyl [4 - {4 - [2 - (2,4 - dichlorophenyl) - 2 - (1 H - imidazol - 1 - ylmethyl) - 1,3 - dioxolan - 4 - ylmethoxy] - phenyl} - 1 - piperazinylcarbonyloxy)acetate and the pharmaceutically acceptable acid addition salts and stereochemically isomeric forms thereof.

4. A composition according to claim 1 wherein said chemical compound is selected from the group consisting of cis - 4 - {4 - [2 - (2,4 - dichlorophenyl) - 2 - (1H - imidazol - 1 - ylmethyl) - 1,3 - dioxolan - 4 - ylmethoxy]phenyl} - N - (2 - methylphenyl) - 1 - piperazineacetamide and the pharmaceutically acceptable acid addition salts and stereochemically isomeric forms thereof.

5. A composition according to claim 1 wherein said chemical compound is selected from the group consisting of cis - N - (2,6 - dichlorophenyl) - 4 - {4 - [2 - (2,4 - dichlorophenyl) - 2 - (1H - imidazol - 1 - ylmethyl) - 1,3 - dioxolan - 4 - ylmethoxy]phenyl} - 1 - piperazineacetamide and the pharmaceutically acceptable acid addition salts and stereochemically isomeric forms thereof.

6. A process for preparing a chemical compound selected from the group consisting of an azole derivative having the formula: 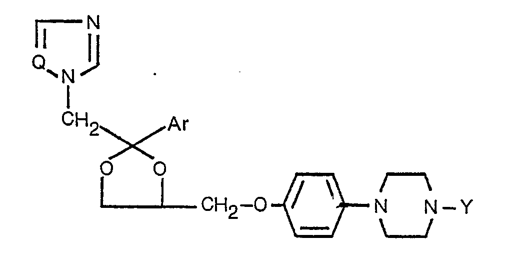 and the pharmaceutically acceptable acid addition salts and stereochemically isomeric forms thereof, wherein Q, Y and Ar are as defined in claim 1 characterised by
a) reacting a compound of the formula 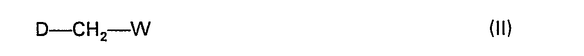 wherein D is 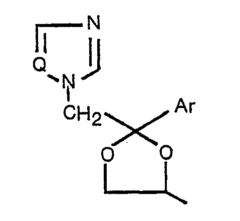 and Q is CH or N and W is a reactive ester residue with a compound of the formula 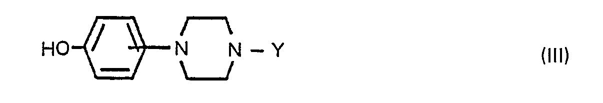 the reaction being carried out in an appropriate reaction-inert organic solvent at elevated temperatures and, if desired, first converting the substituted phenol (III) into a metal salt thereof, and to use thereafter said metal salt in the reaction with (II); or
b) reacting a compound of the formula 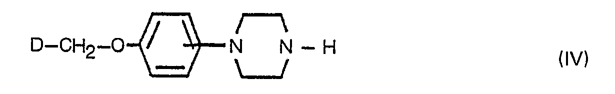 with a compound of the formula 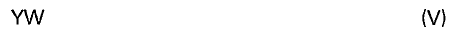 in said reaction the meaning of W as well as the reaction-conditions depending upon the nature of Y;
b) i) in case Y is other than a radical of formula (a), other than a radical of formula (c) wherein n is 0 and other than a radical of formula (c) wherein n is 1 and R³ is C,-C₆ alkyloxycarbonyl C,-C₆ alkyloxy, W may be halo, or a sulfonyloxy group and the reaction is then preferably conducted in a suitable reaction-inert solvent;
b) ii) in case Y represents a radical of formula (a) or a radical of formula (c) wherein n is 0, then W is selected from the group consisting of halo, most preferably chloro, and the reaction is preferably conducted in a suitable reaction-inert solvent; or
c) preparing compounds of formula (I) wherein Y represents a radical (b) wherein alk is as previously defined and wherein R² is as previously defined, but other than cyano and other than aryl, said R² being represented by R²ₐ by substituting the reactive leaving group W in an intermediate of formula 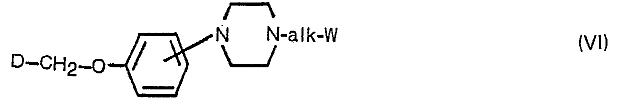 with a compound of the formula 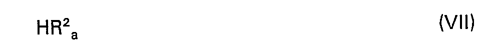 in order to prepare a compound of the formula 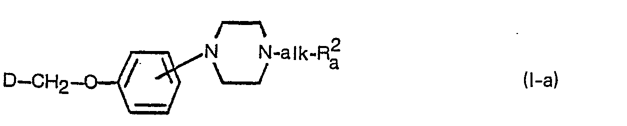 or
c) i) preparing compounds of formula (I-a) wherein R² is amino by reacting (VI) with ammonia wherein 2 protons have been replaced by protective groups, and subsequently eliminating the protective groups following art-known procedures; or
c) ii) preparing primary amine derivatives of formula (I-a) from the corresponding cyanides by reducing the latter following art-known cyano-to-amine reduction procedures such as, catalytic hydrogenation; or
c) iii) preparing compounds of formula (I-a) wherein R²ₐ is amino, mono- and di(C₁―C₆ alkyl)amino, arylamino, mono- and di(aryl C₁―C₆ alkyl)amino, 1-pyrrolidinyl, 1-morpholinyl or 1-piperidinyl starting from an appropriate carbonyl compound and ammonia or an appropriately substituted primary or secondary amine following art-known reductive amination procedures; or
d) preparing the compounds of formula (I) wherein Y represents a radical (d) wherein A and R⁴ are as previously defined and wherein X is 0, by reacting an amine or an alcohol of formula 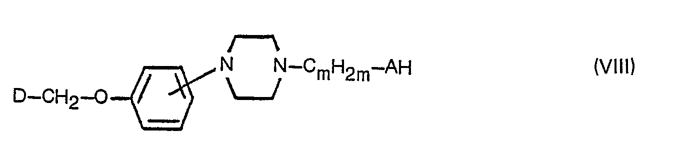 with a compound of the formula 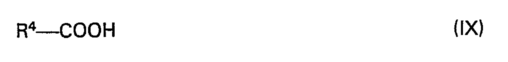 or a functional derivative thereof such as its acyl halide 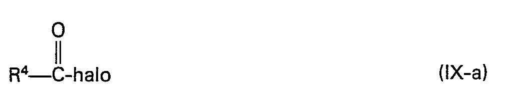 or its anhydride 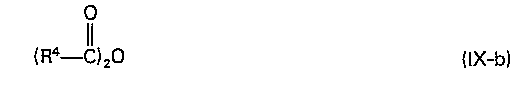 in order to prepare a compound of the formula 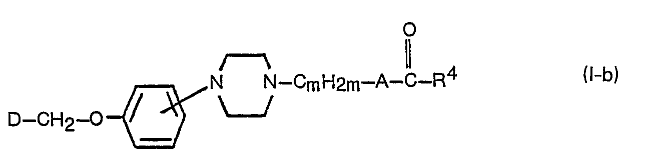 or
d) preparing compounds of formula (I) wherein Y represents a radical (d) wherein A and X are as previously defined and R⁴ is amino, C₁―C₆ alkylamino, arylamino or aryl C₁―C₆ alkylamino, said R⁴ being represented by -NHR⁴ₐ by the addition-reaction of an appropriate amine or alcohol of formula (VIII) with an appropriately substituted isocyanate or isothiocyanate of formula 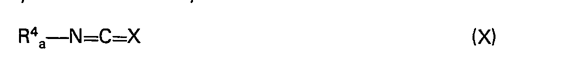 in order to prepare a compound of the formula 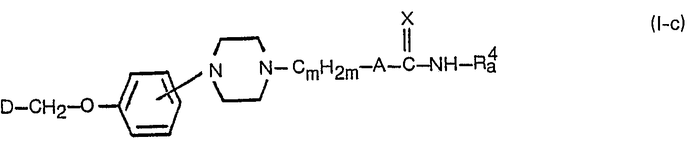 the addition-reaction being preferably carried out by stirring and heating the reactants together in the presence of a suitable reaction-inert solvent; and, if desired, preparing pharmaceutically acceptable acid addition salts or stereochemically isomeric forms of the products thereof.

## Revendications (Revendications pour l'(les) Etat(s) contractant(s) suivant(s) : BE CH DE FR GB IT LU NL SE)

1. Composé chimique choisi parmi le groupe constitué par un dérivé d'azole ayant pour formule: 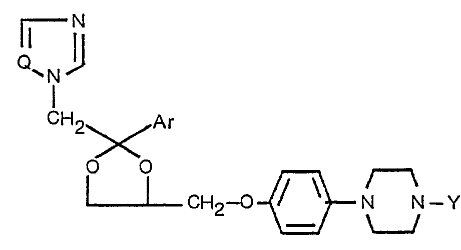 et ses sels d'addition d'acides acceptables en pharmacie et ses formes chimiques stéréo-isomères, où
Q est un constituant choisi dans le groupe composé par N et CH;
Ar est un constituant choisi dans le groupe composé par un phényle, un thiényle, un halogénothiényle et un phényle substitué, ledit phényle substitué ayant de 1 à 3 substituants choisis chacun indépendamment dans le groupe composé par un halogéno, un alkyle en C₁―C₆, un alcoxy en C₁―C₆ et un trifluorométhyle; et
le radical Y est un constituant choisi dans le groupe composé par:
un radical de formule 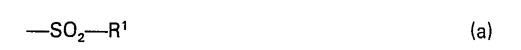 où R' est choisi dans le groupe composé par trifluorométhyle et aryle;
un radical de formule 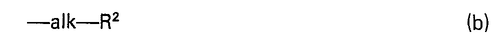 ou Alk est un constituant choisi dans le groupe composé par un alkylène en C₁―C₆ et un alcénylène en C₂―C₆, et
R² est un constituant choisi parmi le groupe composé par un cyano, un amino, un mono- et di-(alkyl en C₁―C₆)amino, un arylamino, un mono- et di-(aryl-alkyl en C₁―C₆)amino, un 1-pyrrolidinyle, un morpholino, un pipéridino, un aryloxy et un aryle, sous réserve que alk soit autre qu'un méthylène lorsque R² est un phényle;
un radical de formule 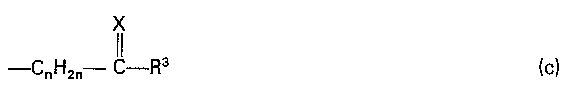 où n est un nombre entier de 0 à 6 inclus,
X est 0 ou S, et
R³ est choisi parmi le groupe composé par un hydrogène, un mono-, di- et trihalogénoalkyle en C₁―C₆, un amino, un mono- et di(alkyl en C₁―C₆)amino, un arylamino, un mono- et di(aryl-alkyl en C₁―C₆)amino, un alkylamino en C₁―C₆, un mono- et di(alkyl en C₁―C₆)aminoalkyle en C₁―C₆, un (1-pyrrolidinyl)alkyle en C₁―C₆, un morpholino-alkyle en C₁―C₆, un pipéridinoalkyle en C₁―C₆, un aryle, un aryl-alkyle en C₁―C₆, un aryl-alcényle en C₂―C₆ et un alcoxy en C₁―C₆ carbonyl-alcoxy en C₁―C₆, sous réserve que
i) ledit n soit autre que 0 ou 1 lorsque ledit R³ est un amino, ou un alkylamino en C₁―C₆; et
ii) ledit n soit autre que 0 lorsque ledit R³ est un hydrogène, un di(alkyl en C₁―C₆)amino ou un aryle; et
un radical de formule 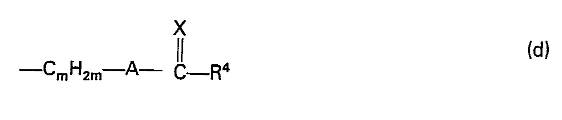 où m est un entier de 1 à 6 inclus,
A est 0 ou NH,
X est 0 ou S, et
R⁴ est un constituant choisi dans le groupe composé par un hydrogène, un alkyle en C₁―C₆, un alcoxy en C₁―C₆, un aryle, un aryloxy, un aryl-alkyle en C₁―C₆, un amino, un mono- et di(alkyl en C₁―C₆)amino, un arylamino, un mono- et di(aryl-alkyl en C₁―C₆)amino, un 1-pyrrolidinyle, un morpholino et un pipéridino;
où ledit aryle, tel qu'on l'emploie dans les définitions précédentes, est choisi parmi le groupe constitué par un phényle, un phényle substitué, un thiényle, un halogénothiényle, un alkyl en C₁―C₆ thiényle et un pyridyle, ledit phényle substitué ayant de 1 à 3 substituants choisis chacun indépendamment dans le groupe constitué par un alkyle en C₁―C₆, un alcoxy en C₁―C₆, un halogéno, un amino, un mono- et un di(alkyl en C₁―C₆)amino, un alkyl en C₁―C₆ carbonylamino, un nitro et un trifluorométhyle.

2. Composé chimique selon la revendication 1, choisi parmi le groupe constitué par le cis - [4 - {4 - [2 - (2,4 - dichlorophényl) - 2 - (1 H - imidazol - 1 - ylméthyl) - 1,3 - dioxolan - 4 - ylméthoxy]phényl} - 1 - pipérazinylcarbonyloxy]acétate d'éthyle et ses sels d'addition d'acides acceptables en pharmacie et ses formes chimiques stéréo-isomères.

3. Composé chimique selon la revendication 1, choisi parmi le groupe constitué par le cis - 4 - {4 - [2 - (2,4 - dichlorophényl) - 2 - (1 H - imidazol - 1 - ylméthyl) - 1,3 - dioxolan - 4 - ylméthoxy]phényl} - N - (2 - méthylphényl) - 1 - pipérazine-acétamide et ses sels d'addition d'acides acceptables en pharmacie et ses formes chimiques stéréo-isomères.

4. Composé chimique selon la revendication 1, choisi parmi le groupe constitué par le cis - N - (2,6 - dichlorophényl) - 4 - {4 - [2 - (2,4 - dichlorophényl) - 2 - (1 H - imidazol - 1 - ylméthyl) - 1,3 - dioxolan - 4 - ylméthoxy]phényl} - 1 - pipérazinyl-acétamide et ses sels d'addition d'acides acceptables en pharmacie et ses formes chimiques stéréo-isomères.

5. Composition pour lutter contre un microorganisme choisi parmi le groupe constitué par les champignons et les bactéries, comprenant une matière support inerte et, comme ingrédient actif, une quantité antifongique ou antibactérienne efficace d'un composé selon l'une quelconque des revendications 1 à 4.

6. Composé chimique selon la revendication 1, choisi parmi le groupe constitué par la cis - 1 - {4 - [2 - (2,4 - dichlorophényl) - 2 - (1 H - imidazol - 1 - ylméthyl) - 1,3 - dioxolan - 4 - ylméthoxy]phényl} - 4 - (2 - pyridylméthyl)pipérazine et ses sels d'addition d'acides acceptables en pharmacie et ses formes chimiques stéréo-isomères.

7. Procédé pour préparer un composé chimique selon la revendication 1, caractérisé par
a) réaction d'un composé de formule 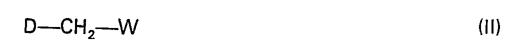 ou D est 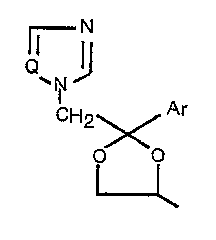
et Q est CH ou N, et
W est un reste d'ester réactif avec un composé de formule 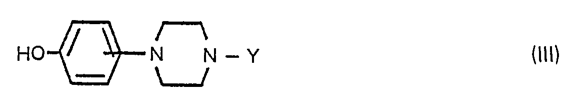 la réaction étant effectuée dans un solvant organique approprié inerte dans la réaction à des températures élevées et, si on le désire, d'abord conversion du phénol substitué (III) en un de ses sels métalliques et emploi ultérieur dudit sel métallique dans la réaction avec (II); ou
b) réaction d'un composé de formule 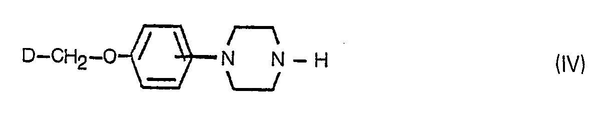 avec un composé de formule YW (V); dans ladite réaction la signification de W ainsi que les conditions réactionnelles dépendant de la nature de Y;
b.i) dans le cas où Y est autre qu'un radical de formule (a), autre qu'un radical de formule (c) où n est 0 et autre qu'un radical de formule (c) où n est 1 et R³ est un alcoxy en C₁―C₆ carbonylalcoxy en C₁―C₆, W peut être un halogèno ou un groupe sulfonyloxy et la réaction est donc de préférence effectuée dans un solvant approprié inerte dans la réaction;
b.ii) dans le cas où Y représente un radical de formule (a) ou un radical de formule (c) où n est 0, W est alors choisi dans le groupe constitué par un halogéno, de façon particulièrement préférable un chloro, et la réaction est de préférence effectuée dans un solvant approprié inerte dans la réaction; ou
c) préparation de composés de formule (I) où Y représente un radical (b) où alk est comme précédemment défini et où R² est comme précédemment défini, mais autre qu'un cyano et autre qu'un aryle, ledit R² étant représenté par R²ₐ par substitution du groupe réactif labile W d'un intermédiaire de formule 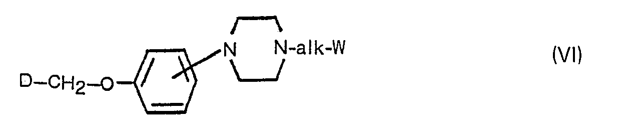 avec un composé de formule 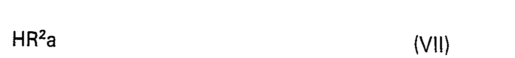 pour préparer un composé de formule 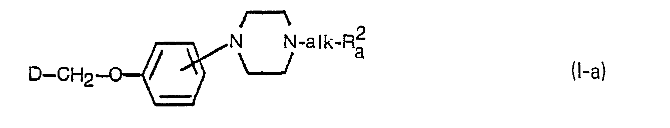 ou
c.i) préparation de composés de formule (I-a) où R²ₐ est un amino par réaction de (VI) avec l'ammoniac où 2 protons ont été remplacés par des groupes protecteurs, puis élimination des groupes protecteurs selon des modes opératoires connus dans l'art; ou
c.ii) préparation de dérivés d'amine primaire de formule (I-a) à partir des cyanures correspondants par réduction de ces derniers selon des modes de réduction de cyano en amine connus dans l'art, tels que l'hydrogénation catalytique; ou
c.iii) préparation de composés de formule (I-a) où R2ₐ est un amino, un mono- et di(alkyl en C₁―C₆)amino, un arylamino, un mono- et di(aryl-alkyl en C₁―C₆)amino, un 1-pyrrolidinyle, un morpholino ou un pipéridino à partir d'un composé carbonylique approprié et d'ammoniac ou d'une amine primaire ou secondaire convenablement substituée selon des opérations d'amination par réduction connues dans l'art;. ou
d) préparation des composés de formule (I) où Y représente un radical (d) où A et R⁴ sont comme précédemment défini et où X et 0, par réaction d'une amine ou d'un alcool de formule 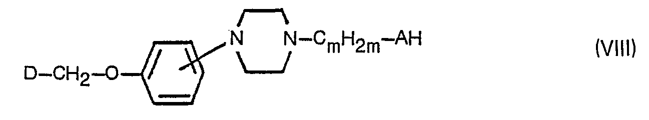 avec un composé de formule: 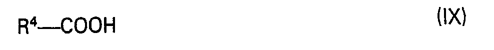 ou un de ses dérivés fonctionnels tels que son halogénure d'acyle 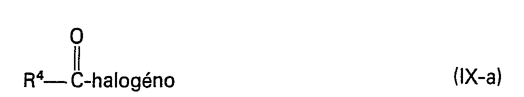 ou son anhydride 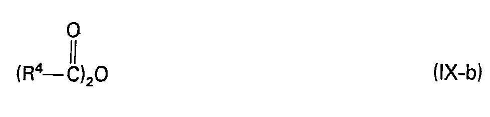 pour préparer un composé de formule 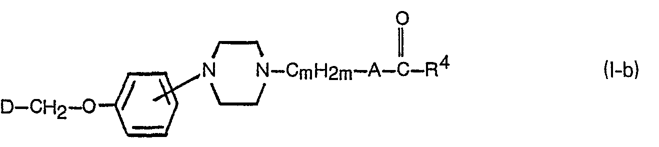 ou
e) préparation de composés de formule (I) où Y représente un radical (d) où A et X sont comme précédemment défini et R⁴ est un amino, un alkylamino en C₁―C₆, un arylamino ou un aryl-alkylamino en C₁―C₆, ledit R⁴ étant représenté par -NHR⁴ₐ par réaction d'addition d'une amine ou d'un alcool appropriés de formule (VIII) avec un isocyanate ou isothiocyanate substitué approprié de formule: 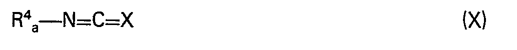 pour préparer un composé de formule 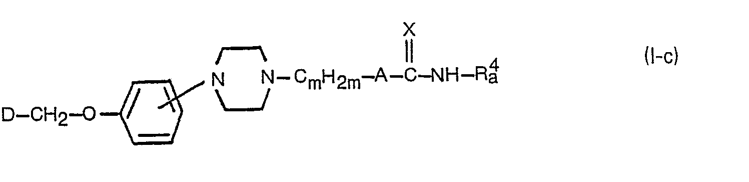 la réaction d'addition étant de préférence effectuée par agitation et chauffage ensemble des composés réagissant en présence d'un solvant approprié inerte dans la réaction;
et, si on le désire, préparation des sels d'addition d'acides acceptables en pharmacie ou des formes chimiques stéréo-isomères des produits.

8. Composition pharmaceutique comprenant une matière support inerte et comme ingrédient actif une quantité antifongique ou antibactérienne efficace d'un composé selon la revendication 6.

9. Composé selon l'une quelconque des revendications 1 à 4 ou 6 ou composition selon la revendication 5 ou la revendication 8, pour l'emploi comme agent antifongique et/ou antibactérien.

## Revendications (Revendications pour l'(les) Etat(s) contractant(s) suivant(s) : AT)

1. Composition pour lutter contre un microorganisme choisi parmi le groupe constitué par les champignons et les bactéries, comprenant une matière support inerte et, comme ingrédient actif, une quantité antifongique ou antibactérienne efficace d'un composé choisi parmi le groupe constitué par un dérivé d'azole ayant pour formule: 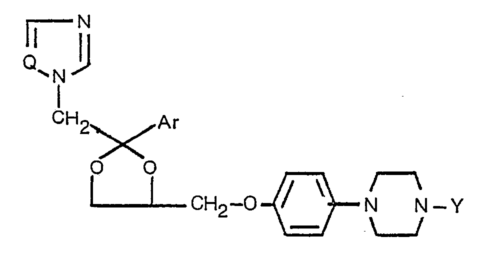 et ses sels d'addition d'acides acceptables en pharmacie et ses formes chimiques stéréo-isomères, où
Q est un constituant choisi dans le groupe composé par N et CH;
Ar est un constituant choisi dans le groupe composé par un phényle, un thiényle, un halogénothiényle et un phényle substitué, ledit phényle substitué ayant de 1 à 3 substituants choisis chacun indépendamment dans le groupe composé par un halogéno, un alkyle en C₁―C₄, un alcoxy en C₁―C₄ et un trifluorométhyle; et
le radical Y est un constituant choisi dans le groupe composé par:
un radical de formule 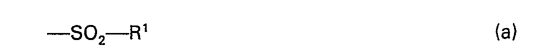 ou R' est choisi dans le groupe composé par trifluorométhyle et aryle;
un radical de formule 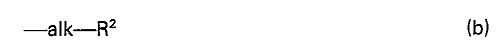
où Alk est un constituant choisi dans le groupe composé par un alkylène en C₁―C₆ et un alcénylène en C₂―C₆, et
R² est un constituant choisi dans le groupe composé par un cyano, un amino, un mono- et di-(alkyl en C₁―C₆)amino, un arylamino, un mono- et di(aryl-alkyl en C₁―C₆)amino, un 1-pyrrolidinyle, un morpholino, un pipéridino, un aryloxy et un aryle, sous réserve que alk soit autre qu'un méthylène lorsque R² est un phényle;
un radical de formule 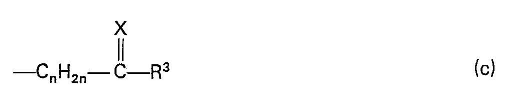 où n est un nombre entier de 0 à 6 inclus,
X est 0 ou S, et ·
R³ est choisi parmi le groupe composé par un hydrogène, un mono-, di- et trihalogénoalkyle en C₁―C₆, un amino, un mono- et di(alkyl en C₁―C₆)amino, un arylamino, un mono- et di(aryl-alkyl en C₁―C₆)amino, un alkylamino en C₁―C₆, un mono- et di(alkyl en C₁―C₆)aminoalkyle en C₁―C₆, un (1-pyrrolidinyl)alkyle en C₁―C₆, un morpholino-alkyle en C₁―C₆, un pipéridinoalkyle en C₁―C₆, un aryle, un aryl-alkyle en C₁―C₆, un aryl-alcényle en C₂―C₆ et un alcoxy en C₁―C₆ carbonyl-alcoxy en C₁―C₆, sous réserve que
i) ledit n soit autre que 0 ou 1 lorsque ledit R³ est un amino, ou un alkylamino en C₁―C₆; et
ii) ledit n soit autre que 0 lorsque ledit R³ est un hydrogène, un di(alkyl en C,-C₆)amino ou un aryle; et
un radical de formule 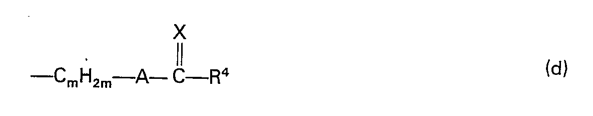 où m est un entier de 1 à 6 inclus,
A est 0 ou NH,
X est 0 ou S, et
R⁴ est un constituant choisi dans le groupe composé par un hydrogène, un alkyle en C₁―C₆, un alcoxy en C₁―C₆, un aryle, un aryloxy, un aryl-alkyle en C₁―C₆, un amino, un mono- et di(alkyl en C₁―C₆)amino, un arylamino, un mono- et di(aryl-alkyl en C₁―C₆)amino, un 1-pyrrolidinyle, un morpholino et un pipéridino;
où ledit aryle, tel qu'on l'emploie dans les définitions précédentes, est choisi parmi le groupe constitué par un phényle, un phényle substitué, un thiényle, un halogénothiényle, un alkyl en C₁―C₆ thiényle et un pyridyle, ledit phényle substitué ayant de 1 à 3 substituants choisis chacun indépendamment dans le groupe constitué par un alkyle en C₁―C₆, un alcoxy en C₁―C₆, un halogéno, un amino, un mono- et un di(alkyl en C₁―C₆)amino, un alkyl en C₁―C₆ carbonylamino, un nitro et un trifluorométhyle.

2. Composition selon la revendication 1, dans laquelle ledit composé chimique est choisi parmi le groupe constitué par le cis - 1 - {4 - [2 - (2,4 - dichlorophényl) - 2 - (1H - imidazol - 1 - ylméthyl) - 1,3 - dioxolan - 4 - yl-méthoxy]phényl} - 4 - (2 - pyridylméthyl)pipérazine et ses sels d'addition d'acides acceptables en pharmacie et ses formes chimiques stéréo-isomères.

3. Composition selon la revendication 1, dans laquelle ledit composé chimique est choisi parmi le groupe constitué par le cis - [4 - {4 - [2 - (2,4 - dichlorophényl) - 2 - (1 H - imidazol - 1 - ylméthyl) - 1,3 - dioxolan - 4 - ylméthoxy]phényl} - 1 - pipérazinylcarbonyloxy acétate d'éthyle et ses sels d'addition d'acides acceptables en pharmacie et ses formes chimiques stéréo-isomères.

4. Composition chimique selon la revendication 1, dans laquelle ledit composé chimique est choisi parmi le groupe constitué par le cis - 4 - {4 - [2 - (2,4 - dichlorophényl) - 2 - (1 H - imidazol - 1 - ylméthyl) - 1,3 - dioxolan - 4 - ylméthoxy]phényl} - N - (2 - méthylphényl) - 1 - pipérazine-acétamide et ses sels d'addition d'acides acceptables en pharmacie et ses formes chimiques stéréo-isomères.

5. Composition selon la revendication 1, dans laquelle ledit composé chimique est choisi parmi le groupe constitué par le cis - N - (2,6 - dichlorophényl) - 4 - (4 - [2 - (2,4 - dichlorophényl) - 2 - (1H - imidazol - 1 - ylméthyl) - 1,3 - dioxolan - 4 - ylméthoxy]phényl} - 1 - pipérazinyl-acétamide et ses sels d'addition d'acides acceptables en pharmacie et ses formes chimiques stéréo-isomères.

6. Procédé pour préparer un composé chimique choisi parmi le groupe constitué par un dérivé d'azole ayant pour formule: 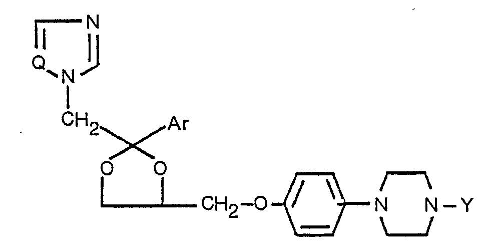 et ses sels d'addition d'acides acceptables en pharmacie et ses formes chimiques stéréo-isomères où Q, Y et Ar sont comme défini dans la revendication 1, caractérisé par:
a) réaction d'un composé de formule 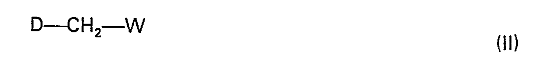 où D est 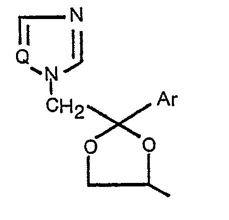 et Q est CH ou N, et
W est un reste d'ester réactif avec un composé de formule 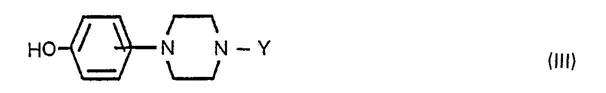 la réaction étant effectuée dans un solvant organique approprié inerte dans la réaction à des températures élevées et, si on le désire, d'abord conversion du phénol substitué (III) en un de ses sels métalliques et emploi ultérieur dudit sel métallique dans la réaction avec (II); ou
b) réaction d'un composé de formule 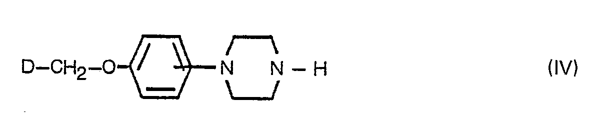 avec un composé de formule YW (V); dans ladite réaction la signification de W ainsi que les conditions réactionnelles dépendant de la nature de Y;
b.i) dans le cas où Y est autre qu'un radical de formule (a), autre qu'un radical de formule (c) ôù n est 0 et autre qu'un radical de formule (c) où n est 1 et R³ est un alcoxy en C₁―C₆ carbonylalcoxy en Cₗ-C₆, W peut être un halogéno ou un groupe sulfonyloxy et la réaction est donc de préférence effectuée dans un solvant approprié inerte dans la réaction;
b.ii) dans le cas où Y représente un radical de formule (a) ou un radical de formule (c) où n est 0, W est alors choisi dans le groupe constitué par un halogéno, de façon particulièrement préférable un chloro, et la réaction est de préférence effectuée dans un solvant approprié inerte dans la réaction; ou
c) préparation de composés de formule (I) où Y représente un radical (b) où alk est comme précédemment défini et où R² est comme précédemment défini, mais autre qu'un cyano et autre qu'un aryle, ledit R² étant représenté par R²ₐ par substitution du groupe réactif labile W d'un intermédiaire de formule 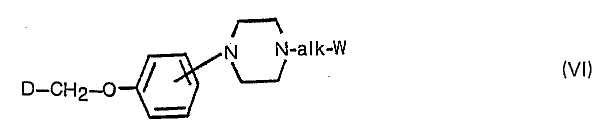 avec un composé de formule 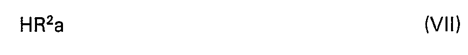 pour préparer un composé de formule 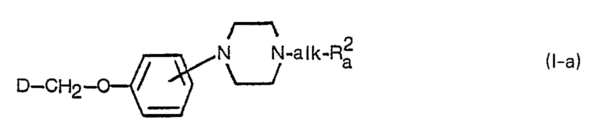 ou
c.i) préparation de composés de formule (I-a) où R²ₐ est un amino par réaction de (VI) avec l'ammoniac où 2 protons ont été remplacés par des groupes protecteurs, puis élimination des groupes protecteurs selon des modes opératoires connus dans l'art; ou
c.ii) préparation de dérivés d'amine primaire de formule (I-a) à partir des cyanures correspondants par réduction de ces derniers selon des modes de réduction de cyano en amine connus dans l'art, tels que l'hydrogénation catalytique; ou
c.iii) préparation de composés de formule (I-a) où R²ₐ est un amino, un mono- et di(alkyl en C₁―C₆)amino, un arylamino, un mono- et di(aryl-alkyl en C₁―C₆)amino, un 1-pyrrolidinyle, un morpholino ou un pipéridino à partir d'un composé carbonylique approprié et d'ammoniac ou d'une amine primaire ou secondaire convenablement substituée selon des opérations d'amination par réduction connues dans l'art; ou
d) préparation des composés de formule (I) où Y représente un radical (d) où A et R⁴ sont comme précédemment défini et où X et 0, par réaction d'une amine ou d'un alcool de formule 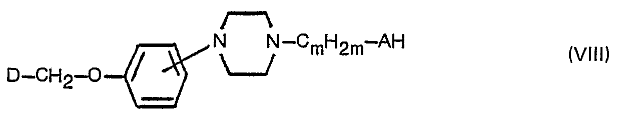 avec un composé de formule: 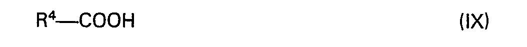 ou un de ses dérivés fonctionnels tels que son halogénure d'acyle 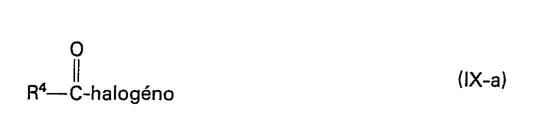 ou son anhydride 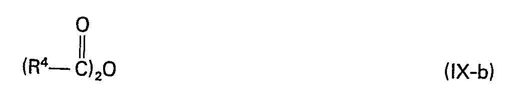 pour préparer un composé de formule 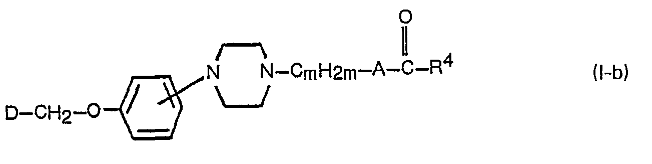 ou
e) préparation de composés de formule (I) où Y représente un radical (d) où A et X sont comme précédemment défini et R⁴ est un amino, un alkylamino en C₁―C₆, un arylamino ou un aryl-alkylamino en C₁―C₆, ledit R⁴ étant représenté par -NHR⁴ₐ par réaction d'addition d'une amine ou d'un alcool appropriés de formule (VIII) avec un isocyanate ou isothiocyanate substitué approprié de formule: 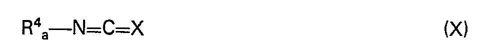 pour préparer un composé de formule 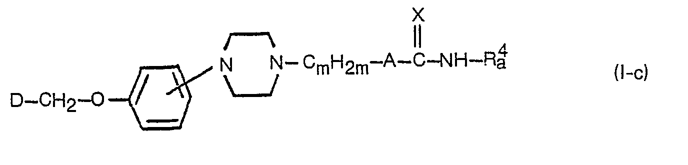 la réaction d'addition étant de préférence effectuée par agitation et chauffage ensemble des composés réagissant en présence d'un solvant approprié inerte dans la réaction;
et, si on le désire, préparation des sels d'addition d'acides acceptables en pharmacie ou des formes chimiques stéréo-isomères des produits.

## Patentansprüche (Patentansprüche für folgende(n) Vertragsstaat(en) : BE CH DE FR GB IT LU NL SE)

1. Eine chemische Verbindung, ausgewählt aus der Gruppe, die aus einem Azolderivat der Formel 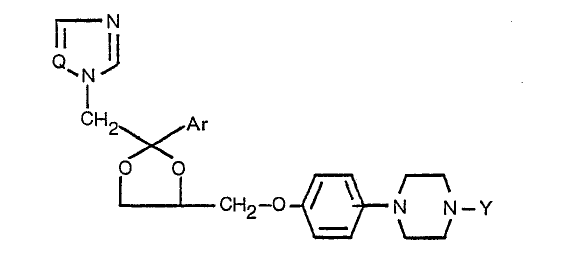 und den pharmazeutisch annehmbaren Säureadditionssalzen und stereochemisch isomeren Formen hievon besteht, worin:
Q ein Vertreter aus der N und CH umfassenden Gruppe ist;
Ar ein Vertreter aus der Gruppe ist, die aus Phenyl, Thienyl, Halogenthienyl und substituertem Phenyl besteht, wobei das genannte substituierte Phenyl 1 bis 3 Substituenten aufweist, die jeweils unabhängig voneinander ausgewählt sind aus der aus Halogen, C₁―C₆-Alkyl, C₁―C₆-Alkyloxy und Trifluormethyl umfassenden Gruppe; und
der Rest Y ein Vertreter aus der Gruppe ist, die besteht aus:
einem Rest der Formel 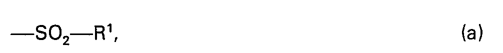 worin R' ausgewählt ist aus Trifluormethyl und Aryl;
einem Rest der Formel 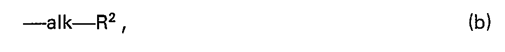 worin alk ein Vertreter, ausgewählt aus der aus C₁―C₆-Alkylen und C₂―C₆-Alkenylen bestehenden Gruppe, ist und R² ein Vertreter aus der aus Cyano, Amino, Mono- und Di(C₁―C₆-alkyl)amino, Arylamino, Mono- und di-(aryl C₁―C₆ alkyl)amino, 1-Pyrrolidinyl, 1-Morpholinyl, 1-Piperidinyl, Aryloxy und Aryl bestehenden Gruppe ist, mit der Maßgabe, daß alk eine andere Bedeutung als Methylen aufweist, wenn R² für Phenyl steht;
einem Rest der Formel 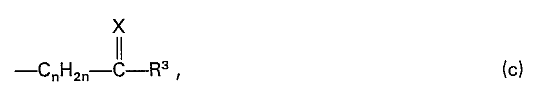 worin n eine ganze Zahl von 0 bis einschließlich 6 bedeutet, X für Sauerstoff oder Schwefel steht und R³ ausgewählt ist aus der Gruppe, bestehend aus Wasserstoff, Mono-, Di- und Trihalogen-C₁―C₆-alkyl, Amino, Mono- und Di(C₁―C₆ alkyl)amino, Arylamino, Mono- und Di(aryl-C₁―C₆-alkyl)amino, Amino-C₁―C₆-alkyl, Mono- und Di(C₁―C₆-alkyl)amino-C₁―C₆-alkyl; (1-Pyrrolidinyl)C₁―C₆-alkyl, (1-Morpholinyl)C₁―C₆-alkyl, (1-Piperidinyl)C₁―C₆-alkyl, Aryl, Aryl-C₁―C₆-alkyl, Aryl-C₂―C₆-alkenyl und C₁―C₅-Alkyloxycarbonyl-C₁―C₅-alkyloxy, mit der Maßgabe, daß:
i) genanntes n einen anderen Wert als 0 oder 1 aufweist, wenn der erwähnte Rest R³ Amino oder C₁―C₆-Alkylamino bedeutet; und
ii) genanntes n einen anderen Wert als 0 aufweist, wenn der erwähnte Rest R³ Wasserstoff, Di(C₁―C₆-alkyl)amino oder Aryl darstellt; und
einem Rest der Formel 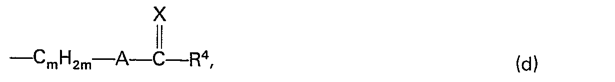 worin m eine ganze Zahl von 1 bis einschließlich 6 bedeutet, A für Sauerstoff oder NH steht, X für Sauerstoff oder Schwefel steht und R⁴ ein Vertreter aus einer Gruppe, bestehend aus Wasserstoff, C₁―C₆-Alkyl, C₁―C₆Alkyloxy, Aryl, Aryloxy, Aryl-C₁―C₆-alkyl, Amino, Mono- und Di(C₁―C₆- alkyl)amino, Arylamino, Mono- und Di(aryl-C₁―C₆-alkyl)amino, 1-Pyrrolidinyl, 1-Morpholinyl und 1-Piperidinyl, ist;
worin in den vorstehenden Definitionen genanntes Aryl ausgewählt ist aus der Gruppe umfassend Phenyl, substituiertes Phenyl, Thienyl, Halogenthienyl, C₁―C₆-Alkylthienyl und Pyridinyl, wobei das genannte substituierte Phenyl 1 bis 3 Substituenten aufweist, von denen jeder unabhängig voneinander ausgewählt ist aus einer C₁―C₆Alkyl, C₁―C₆-Alkyloxy, Halogen, Amino, Mono- und Di-(C₁―C₆ alkyl)amino, C₁―C₆-Alkylcarbonylamino, Nitro und Trifluormethyl umfassenden Gruppe.

2. Eine chemische Verbindung gemäß Anspruch 1, ausgewählt aus der cis - Ethyl - [4 - {4 - [2 - (2,4 - dichlorphenyl) - 2 - (1 H - imidazol - 1 - ylmethyl) - 1,3 - dioxolan - 4 - ylmethoxy]-phenyl} - 1 - piperazinylcarbonyloxy]acetat und die pharmazeutisch annehmbaren Säureadditionssalze und stereochemisch isomeren Formen hievon umfassenden Gruppe.

3. Eine chemische Verbindung gemäß Anspruch 1, ausgewählt aus der cis - 4 - {4 - [2 - (2,4 - Dichlorphenyl) - 2 - (1H - imidazol - 1 - ylmethyl) - 1,3 - dioxolan - 4 - ylmethoxy]phenyl} - N - (2 - methylphenyl) - 1 - piperazinacetamid und die pharmazeutisch annehmbaren Säureadditionssalze und stereochemisch isomeren Formen hievon umfasenden Gruppe.

4. Eine chemische Verbindung gemäß Anspruch 1, ausgewählt aus der cis - N - (2,6-Dichlorphenyl) - 4 - {4 - [2 - (2,4 - dichlorphenyl) - 2 - (1 H - imidazol - 1 - ylmethyl) - 1,3 - dioxolan - 4 - ylmethoxy]phenyl} - 1 - piperazinacetamid und die pharmazeutisch annehmbaren Säureadditionssalze und stereochemisch isomeren Formen hievon umfassenden Gruppe.

5. Eine Zusammensetzung zur Bekämpfung eines Mikroorganismus, ausgewählt aus der aus Fungus und Bakterium bestehenden Gruppe, umfassend ein inertes Trägermaterial und als einen wirksamen Bestandteil eine antifungal oder antibakteriell wirksame Menge einer Verbindung gemäß einem der Ansprüche 1 bis 4. -

6. Eine chemische Verbindung gemäß Anspruch 1, ausgewählt aus der cis - 1 - {4 - [2 - (2,4 - Dichlorphenyl) - 2 - (1H - imidazol - 1 - ylmethyl) - 1,3 - dioxolan - 4 - ylmethoxy]phenyl} - 4 - (2 - pyridinylmethyl)piperazin und die pharmazeutisch annehmbaren Säureadditionssalze und stereochemisch isomeren Formen hievon umfassenden Gruppe.

7. Ein Verfahren zur Herstellung einer chemischen Verbindung gemäß Anspruch 1, gekennzeichnet durch
a) Umsetzen einer Verbindung der formel 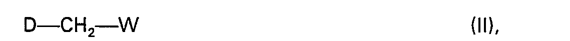 worin D für 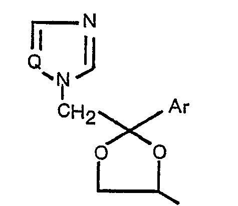 steht und Q für CH oder N steht einen reaktionsfähigen Esterrest bedeutet, mit einer Verbindung der Formel 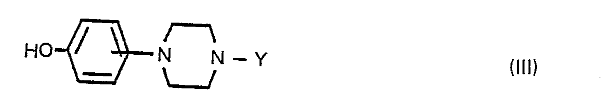 wobei die Umsetzung in einem geeigneten reaktions-inerten organischen Lösungsmittel bei erhöhten Temperaturen ausgeführt und gewünschtenfalls zunächst das substituierte Phenol (III) in ein Metallsalz hievon übergeführt und hierauf dieses Metallsalz in der Umsetzung mit (II) verwendet wird; oder
b) Umsetzen einer Verbindung der Formel 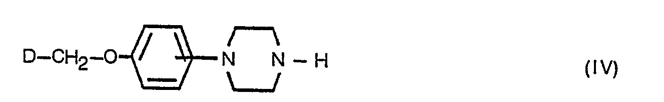 mit einer Verbindung der Formel 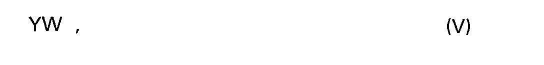 wobei in. der genannten Umsetzung sowohl die Bedeutung von W als auch die Reaktionsbedingungen von der Art von Y abhängen;
b) i) für den Fall, daß Y eine andere Bedeutung als einen Rest der Formel (a), eine andere Bedeutung als einen Rest der Formel (c), worin n den Wert 0 hat, und eine andere Bedeutung als einen Rest der Formel (c), worin n den Wert 1 hat und R³ für C₁―C₆-Alkyloxycarbonyl-C₁―C₆-alkyloxy steht, aufweist, W für Halogen oder eine Sulfonyloxygruppe steht und die Reaktion dann vorzugsweise in einem geeigneten reaktionsinerten Lösungsmittel ausgeführt wird;
b) ii) für den Fall, daß Y einen Rest der Formel (a) oder einen Rest der Formel (c), worin n den Wert 0 hat, darstellt, dann W ausgewählt wird aus der aus Halogen bestehenden Gruppe, am meisten bevorzugt Chlor, und die Reaktion vorzugsweise in einem geeigneten reaktions-inerten Lösungsmittel ausgeführt wird; oder
c) Bereiten von Verbindungen der Formel (1), worin Y einen Rest (b) darstellt, worin alk wie zuvor definiert ist und worin R² wie zuvor definiert ist, jedoch eine andere Bedeutung als Cyano und eine andere Bedeutung als Aryl aufweist, welches R² durch R²ₐ dargestellt wird, durch Ersetzen der reaktionsfähigen Leaving-Gruppe W in einem Zwischenprodukt der Formel 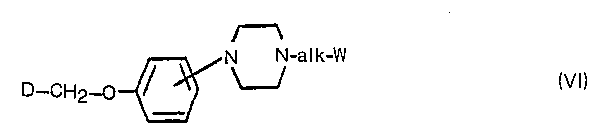 durch eine Verbindung der Formel 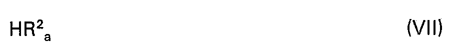 zur Bildung einer Verbindung der Formel 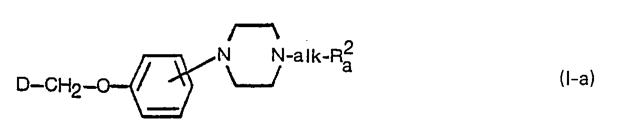 oder
c) i) Bereiten von Verbindungen der Formel (I-a), worin R²ₐ Amino bedeutet, durch Umsetzen von (VI) mit Ammoniak, worin 2 Protonen durch Schutzgruppen ersetzt worden sind, und anschließendes Abtrennen der Schutzgruppen nach bekannten Methoden; oder
c) ii) Bereiten von primären Aminderivaten der Formel (I-a) aus den entsprechenden Cyaniden durch Reduzieren der letztgenannten nach bekannten Cyan-zu-Amin-Reduktions-verfahren, wie katalytische Hydrierung; oder
c) iii) Bereiten von Verbindungen der Formel (I-a), worin R² für Amino, Mono- und Di(C₁―C₆- alkyl)amino, Arylamino, Mono- und Di(aryl-C₁―C₆-alkyl)amino, 1-Pyrrolidinyl, 1-Morpholinyl oder 1-Piperidinyl steht, aus einer geeigneten Carbonylverbindung und Ammoniak oder einem geeignet substituierten primären oder sekundären Amin nach bekannten reduktiven Aminierungsverfahren; oder
d) Bereiten von Verbindungen der Formel (I), worin Y einen Rest (d) darstellt, worin A und R⁴ wie zuvor definiert sind und worin X für Sauerstoff steht, durch Umsetzen eines Amins oder eines Alkohols der Formel 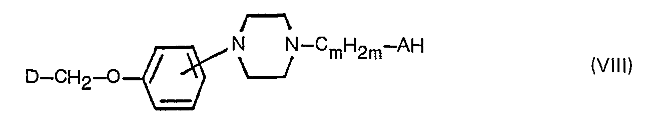 mit einer Verbindung der Formel 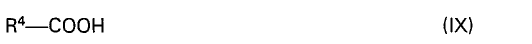 oder einem funktionellen Derivat hievon wie dessen Acylhalogenid 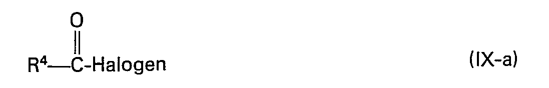 oder dessen Anhydrid 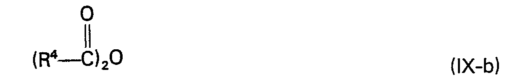 zur Ausbildung einer Verbindung der Formel 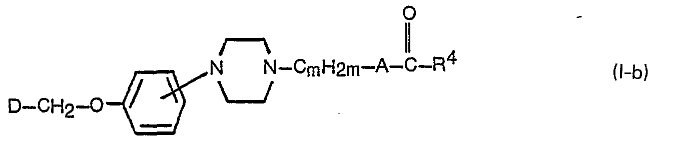 oder
e) Bereiten von Verbindungen der Formel (I), worin Y einen Rest (d) darstellt, worin A und X wie zuvor definiert sind und R⁴ für Amino, C₁―C₆-Alkylamino, Arylamino oder Aryl-C₁―C₆-alkylamino steht, welches R⁴ durch -NHR⁴ₐ dargestellt wird, durch die Additionsreaktion eines geeigneten Amins oder Alkohols der Formel (VIII) mit einem geeignet substituierten Isocyanat oder Isothiocyanat der Formel 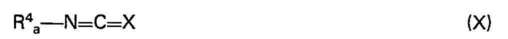 zur Ausbildung einer Verbindung der Formel 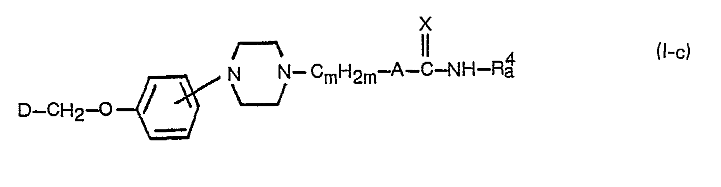 welche Additionsreaktion vorzugsweise durch gemeinsames Rühren und Erhitzen der Reaktionspartner in Gegenwart eines geeigneten reaktions-inerten Lösungsmittels ausgeführt wird;
und gewünschtenfalls Herstellen von pharmazeutisch annehmbaren Säureadditionssalzen oder stereochemisch isomeren Formen der Produkte hievon.

8. Eine pharmazeutische Zusammensetzung, umfassend ein inertes Trägermaterial und als einen wirksamen Bestandteil eine antifungal oder antibakteriell wirksame Menge einer Verbindung gemäß Anspruch 6.

9. Eine Verbindung gemäß einem der Ansprüche 1 bis 4 oder 6, oder eine Zusammensetzung gemäß Anspruch 5 oder 8, zur Verwendung als antifungales und/oder antibakterielles Mittel.

## Patentansprüche (Patentansprüche für folgende(n) Vertragsstaat(en) : AT)

1. Eine Zusammensetzung zur Bekämpfung eines Mikroorganismus, ausgewählt aus der aus Fungus und Bacterium bestehenden Gruppe, umfassend ein inertes Trägermaterial und als einen wirksamen Bestandteil eine antifungal oder antibakteriell wirksame Menge einer Verbindung, ausgewählt aus der Gruppe, die aus einem Azolderivat der Formel 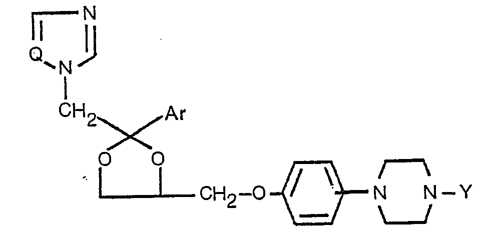 und den pharmazeutisch annehmbaren Säureadditionssalzen und stereochemisch isomeren Formen hievon besteht, worin:
Q ein Vertreter aus der N und CH umfassenden Gruppe ist;
Ar ein Vertreter aus der Gruppe ist, die aus Phenyl, Thienyl, Halogenthienyl und substituertem Phenyl besteht, wobei das genannte substituierte Phenyl 1 bis 3 Substituenten aufweist, die jeweils unabhängig voneinander ausgewählt sind aus der aus Halogen, C₁―C₆-Alkyl, C₁―C₆-Alkyloxy und Trifluormethyl umfassenden Gruppe; und
der Rest Y ein Vertreter aus der Gruppe ist, die besteht aus:
einem Rest der Formel 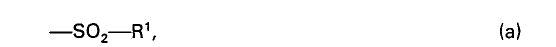 worin R' ausgewählt ist aus Trifluormethyl und Aryl;
einem Rest der Formel 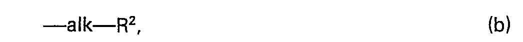 worin alk ein Vertreter, ausgewählt aus der aus C₁―C₆-Alkylen und C₂―C₆-Alkenylen bestehenden Gruppe, ist und R² ein Vertreter aus der aus Cyano, Amino, Mono- und Di(C₁―C₆-alkyl)amino, Arylamino, Mono- und Di-(aryl C₁―C₆ alkyl)amino, 1-Pyrrolidinyl, 1-Morpholinyl, 1-Piperidinyl, Aryloxy und Aryl bestehenden Gruppe ist, mit der Maßgabe, daß alk eine andere Bedeutung als Methylen aufweist, wenn R² für Phenyl steht;
einem Rest der Formel 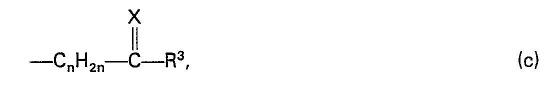 worin n eine ganze Zahl von 0 bis einschließlich 6 bedeutet, X für Sauerstoff oder Schwefel steht und R³ ausgewählt ist aus der Gruppe, bestehend aus Wasserstoff, Mono-, Di- und Trihalogen-C₁―C₆-alkyl, Amino, Mono- und Di(C₁―C₆ alkyl)amino, Arylamino, Mono- und Di(aryl-C₁―C₆-alkyl)amino, Amino-C₁―C₆-alkyl, Mono- und Di(C₁―C₆-alkyl)amino-C₁―C₆-alkyl, (1-Pyrrolidinyl)C₁―C₆-alkyl, (1-Morpholinyl)C₁―C₆-alkyl, (1-Piperidinyl)C₁―C₆-alkyl, Aryl, Aryl-C₁―C₆-alkyl, Aryl-C₂―C₆-alkenyl und C₁―C₆-Alkyloxycarbonyl-C₁―C₆-alkyloxy, mit der Maßgabe, daß:
i) genanntes n einen anderen Wert als 0 oder 1 aufweist, wenn der erwähnte Rest R³ Amino oder C₁―C₆-Alkylamino bedeutet; und
ii) genanntes n einen anderen Wert als 0 aufweist, wenn der erwähnte Rest R³ Wasserstoff, Di(C₁―C₆-alkyl)amino oder Aryl darstellt; und
einem Rest der Formel 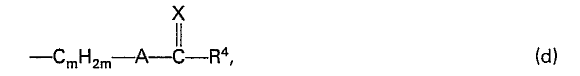 worin m eine ganze Zahl von 1 bis einschließlich 6 bedeutet, A für Sauerstoff oder NH steht, X für Sauerstoff oder Schwefel steht und R⁴ ein Vertreter aus einer Gruppe, bestehend aus Wasserstoff, C₁―C₆-Alkyl, C₁―C₆-Alkyloxy, Aryl, Aryloxy, Aryl-C₁―C₆-alkyl; Amino, Mor und Di(C₁―C₆- alkyl)amino, Arylamino, Mono- und Di(aryl-C₁―C₆-alkyl)amino, 1-Pyrrolidinyl, 1-, porpholinyl und 1-Piperidinyl, ist;
worin in den vorstehenden Definitionen genanntes Aryl ausgewählt ist aus der 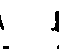 uppe umfassend Phenyl, substituiertes Phenyl, Thienyl, Halogenthienyl, C₁―C₆-Alkylthienyl und Pyridi, 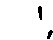 , wobei das genannte substituierte Phenyl 1 bis 3 Substituenten aufweist, von denen jeder unabhä, 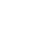 ig voneinander ausgewählt ist aus einer C₁―C₆-Alkyl, C₁―C₆-Alkyloxy, Halogen, Amino, Mono- un kyl)amino, C₁―C₆-Alkylcarbonylamino, Nitro und Trifluormethyl umfassenden Gruppe.

2. Eine Zusammensetzung gemäß Anspruch 1, worin die genannte chemische Verbindung ausgewählt ist aus der cis - 1 - (4 - [2 - (2,4 - Dichlorphenyl) - 2 - (1 H - imidazol - 1 - ylmethyl) - 1,3 - dioxolan - 4 - ylmethoxy]phenyl} - 4 - (2 - pyridinylmethyl)piperazin und die pharmazeutisch annehmbaren Säureadditionssalze und stereochemisch isomeren Formen hievon umfassenden Gruppe.

3. Eine Zusammensetzung gemäß Anspruch 1, worin die genannte chemische Verbindung aus gewählt ist aus der cis - Ethyl - [4 - {4 - [2 - (2,4 - dichlorphenyl) - 2 - (1H - imidazol - 1 - ylmethyl) - 1,3 - dioxolan - 4 - ylmethoxy]phenyl} - 1 - piperazinylcarbonyloxy]acetat und die pharmazeutisch annehmbaren Säureadditionssalze und stereochemisch isomeren Formen hievon umfassenden Gruppe.

4. Eine Zusammensetzung gemäß Anspruch 1, worin die genannte chemische Verbindung ausgewählt ist aus der cis - 4 - {4 - [2 - (2,4 - Dichlorphenyl) - 2 - (1H - imidazol - 1 - ylmethyl) - 1,3 - dioxolan - 4 - ylmethoxy]phenyl} - N - (2 - methylphenyl) - 1 - piperazinacetamid und die pharmazeutisch annehmbaren Säureadditionssalze und stereochemisch isomeren Formen hievon umfassenden Gruppe

5. Eine Zusammensetzung gemäß Anspruch 1, worin die genannte chemische Verbindung ausgewählt ist aus der cis - N - (2,6-Dichlorphenyl) - 4 - {4 - [2 - (2,4 - dichlorphenyl) - 2 - (1 H - imidazol - 1 - ylmethyl) - 1,3 - dioxolan - 4 - ylmethoxy]phenyl} - 1 - piperazinacetamid und die pharmazeutisch annehmbaren Säureadditionssalze und stereochemisch isomeren Formen hievon umfassenden Gruppe.

6. Ein Verfahren zur Herstellung einer chemischen Verbindung, ausgewählt aus der Gruppe, die aus einem Azolderivat mit der Formel: 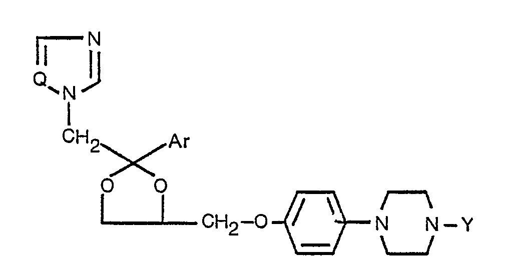 und den pharmazeutisch annehmbaren Säureadditionssalzen und stereochemisch isomeren Formen hievon besteht, worin Q, Ar und Y wie in Anspruch 1 definiert sind, gekennzeichnet durch
a) Umsetzen einer Verbindung der formel 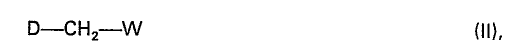 worin D für 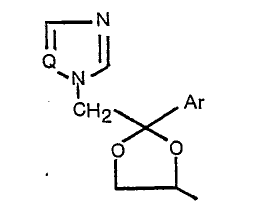 steht und Q für CH oder N steht und W einen Reaktionsfähigen Esterrest bedeutet, mit einer Verbindung der Formel 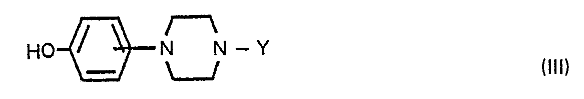 wobei die Umsetzung in einem geeigneten ᵣₑₐₖtiₒₙₛ-inerten organischen Lösungsmittel bei erhöhten Temperaturen ausgeführt und gewünschtenfalls zunächst das substituierte Phenol (III) in ein Metallsalz hievon übergeführt und hierauf dieses Metallsalz in der Umsetzung mit (11) verwendet wird; oder
b) Umsetzen einer Verbindung der Formel 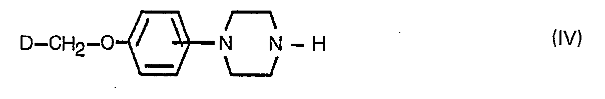 mit einer Verbindung der Formel YW 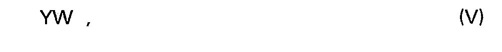 wobei in der genannten Umsetzung sowohl die Bedeutung von W als auch die Reaktionsbedingungen von der Art von Y abhängen;
b) i) für den Fall, daß Y eine andere Bedeutung als einen Rest der Formel (a), eine andere Bedeutung als einen Rest der Formel (c), worin n den Wert 0 hat, und eine andere Bedeutung als einen Rest der Formel (c), worin n den Wert 1 hat und R³ für C₁―C₆-Alkyloxycarbonyl-C₁―C₆-alkyloxy steht, aufweist, W für Halogen oder eine Sulfonyloxygruppe steht und die Reaktion dann vorzugsweise in einem geeigneten reaktionsinerten Lösungsmittel ausgeführt wird;
b) ii) für den Fall, daß Y einen Rest der Formel (a) oder einen Rest der Formel (c), worin n den Wert 0 hat, darstellt, dann W ausgewählt wird aus der aus Halogen bestehenden Gruppe, am meisten bevorzugt Chlor, und die Reaktion vorzugsweise in einem geeigneten reaktions-inerten Lösungsmittel ausgeführt wird; oder
c) Bereiten von Verbindungen der Formel (I), worin Y einen Rest (b) darstellt, worin alk wie zuvor definiert ist und worin R² wie zuvor definiert ist, jedoch eine andere Bedeutung als Cyano und eine andere Bedeutung als Aryl aufweist, welches R² durch R²ₐ dargestellt wird, durch Ersetzen der reaktionsfähigen Leaving-Gruppe W in einem Zwischenprodukt der Formel 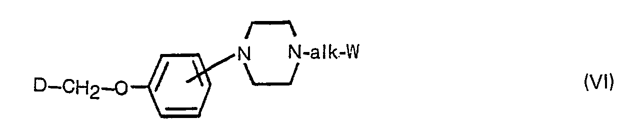 durch eine Verbindung der Formel 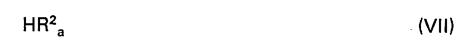 zur Bildung einer Verbindung der Formel 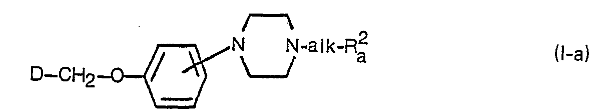 oder
c) i) Bereiten von Verbindungen der Formel (I-a), worin R²ₐ Amino bedeutet, durch Umsetzen von (VI) mit Ammoniak, worin 2 Protonen durch Schutzgruppen ersetzt worden sind, und anschließendes Abtrennen der Schutzgruppen nach bekannten Methoden; oder
c) ii) Bereiten von primären Aminderivaten der Formel (I-a) aus den entsprechenden Cyaniden durch Reduzieren der letztgenannten nach bekannten Cyan-zu-Amin-Reduktions-verfahren, wie katalytische Hydrierung; oder
c) iii) Bereiten von Verbindungen der Formel (I-a), worin R²ₐ für Amino, Mono- und Di(C₁―C₆- alkyl)amino, Arylamino, Mono- und Di(aryl-C₁―C₆-alkyl)amino, 1-Pyrrolidinyl, 1-Morpholinyl oder 1-Piperidinyl steht, aus einer geeigneten Carbonylverbindung und Ammoniak oder einem geeignet substituierten primären oder sekundären Amin nach bekannten reduktiven Aminierungsverfahren; oder
d) Bereiten von Verbindungen der Formel (I), worin Y einen Rest (d) darstellt, worin A und R⁴ wie zuvor definiert sind und worin X für Sauerstoff steht, durch Umsetzen eines Amins oder eines Alkohols der Formel 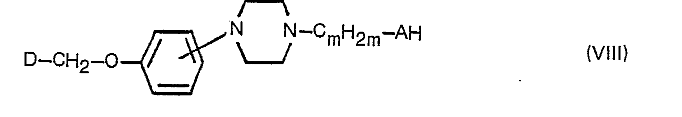 mit einer Verbindung der Formel 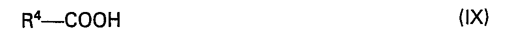 oder einem funktionellen Derivat hievon wie dessen Acylhalogenid 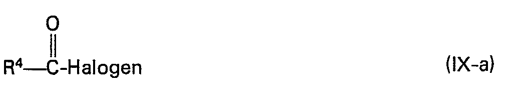 oder dessen Anhydrid 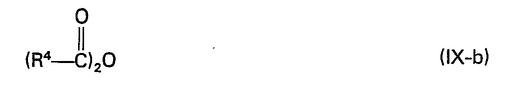 zur Ausbildung einer Verbindung der Formel 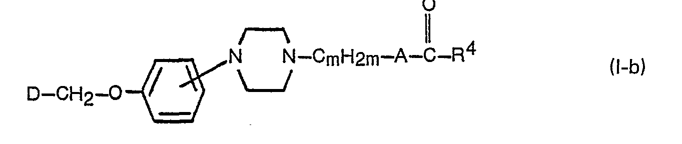 oder
e) Bereiten von Verbindungen der Formel (1), worin Y einen Rest (d) darstellt, worin A und X wie zuvor definiert sind und R⁴ für Amino, C₁―C₆-Alkylamino, Arylamino oder Aryl-C₁―C₆-alkylamino steht, welches R⁴ durch ―NHR⁴ₐ dargestellt wird, durch die Additionsreaktion eines geeigneten Amins oder Alkohols der Formel (VIII) mit einem geeignet substituierten Isocyanat oder Isothiocyanat der Formel 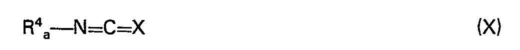 zur Ausbildung einer Verbindung der Formel 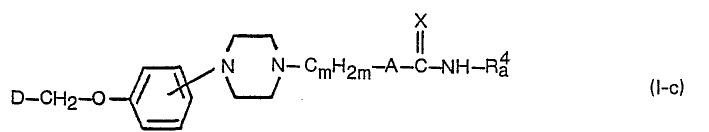 welche Additionsreaktion vorzugsweise durch gemeinsames Rühren und Erhitzen der Reaktionspartner in Gegenwart eines geeigneten reaktions-inerten Lösungsmittels ausgeführt wird;
und gewünschtenfalls Herstellen von pharmazeutisch annehmbaren Säureadditionssalzen oder stereochemisch isomeren Formen der Produkte hievon.
